# EUROPEAN PATENT APPLICATION

(11) **EP 4 578 449 A1**
(43) Date of publication of application: **02.07.2025**
(21) Application number: 23856617.8
(22) Date of filing: 22.08.2023
(51) Int. Cl.: A61K 31/495, A61K 31/40, A61K 39/395, A61P 35/00

(54) **PHARMACEUTICAL COMBINATION OF FAK INHIBITOR AND MICROTUBULE INHIBITOR AND USE THEREOF**

(30) Priority: 24.08.2022 CN 202211018821; 23.09.2022 CN 202211165722; 28.07.2023 CN 202310940775
(71) Applicant: Inxmed (Nanjing) Co., Ltd., Nanjing, Jiangsu 210061 (CN)
(72) Inventor: ZHANG, Baoyuan, Nanjing, Jiangsu 210061 (CN); LIU, Xuebin, Nanjing, Jiangsu 210061 (CN); GAO, Jiaming, Nanjing, Jiangsu 210061 (CN); ZHANG, Ping, Nanjing, Jiangsu 210061 (CN); PANG, Ran, Nanjing, Jiangsu 210061 (CN); WANG, Zaiqi, Nanjing, Jiangsu 210061 (CN)
(74) Representative: D Young & Co LLP
(86) International application number: PCT/CN2023/114225
(87) International publication number: WO 2024/041527

(57) **Abstract**

Provided are a composition of an FAK inhibitor, a microtubule inhibitor and an immune checkpoint inhibitor, a kit composed of the composition, and use thereof in the preparation of a medicament for treating a tumor.

## Description

This application claims priority to Chinese patent application 202211018821.9 filed on August 24, 2022, Chinese patent application 202211165722.3 filed on September 23, 2022, and Chinese patent application 202310940775.6 filed on July 28, 2023. The disclosures of the above-mentioned Chinese patent applications are cited in their entirety as part of this application.

### TECHNICAL FIELD

The present disclosure belongs to the field of medicinal chemistry. Specifically, the present disclosure relates to the treatment of a tumor using a focal adhesion kinase (FAK) inhibitor in combination with other drugs.

### BACKGROUND

Tumor is the second largest killer threatening people's health. Immunogenic cell death (ICD) is based on the superposition effect of programmed cell death. An intracellular pressure signal may be activated when cancer cells are exposed to chemotherapy or targeted medicaments. This kind of pressure signal includes endoplasmic reticulum pressure (ER stress) and reactive oxygen species pressure (oxidative stress). Under the action of the pressure signal, cells will try to repair the pressure first. The cells will start the programmed death process, if the damage caused by the pressure exceeds the repair ability of the cells. This process is often accompanied by the release of a class of damage associated molecular patterns (DAMPs), which include calreticulin, annexin A1, HMGB1, etc. This kind of DAMPs will be specifically recognized by pattern recognition receptors on antigen-presenting cells (APC) in the body, which will induce the maturation, differentiation and activation of APC, and then gradually present it to immune cells such as effector T cells, thus making immune cells generate antigen memory. When tumor cells from the same source are found again, immune cells will specifically recognize and kill the tumor cells. The new tumor-specific immune response initiated by ICD can increase the sensitivity to an immune checkpoint inhibitor (ICI), so as to enhance the effect of the immune checkpoint inhibitor, and produce anti-tumor response with lasting immune memory.

FAK, also known as protein tyrosine kinase 2 (PTK2), is a non-receptor tyrosine kinase and a key component of focal adhesion complex. FAK plays an important role in mediating integrin and growth factor signals to regulate the invasion, proliferation and survival of tumor cells.

Microtubules are a dynamic filamentous cytoskeletal protein and the main molecule of centrioles. The orderly dynamic changes of microtubule fibers are an important guarantee for cell mitosis. Microtubule inhibitors bind to tubulin to promote or inhibit the assembly of microtubules, to interfere with the normal structure and function of the spindle composed of microtubules, to interfere with cell mitosis, to inhibit cell proliferation, and to exert clear anti-tumor effects. Anti-tumor drugs targeting microtubules have become an important aspect of current anti-tumor drug research. There are currently many microtubule inhibitors on the market, including taxanes and Eribulin. Taxane drugs include paclitaxel extracted from natural tree bark, synthetic docetaxel, and now albumin paclitaxel, etc. Taxane drugs are M-phase cycle-specific drugs that can promote the polymerization of tubules into stable microtubules and inhibit their depolymerization, thereby significantly reducing the number of tubules and destroying the microtubule network structure. Eribulin is a synthetic analog of halichondrin B, which inhibits microtubule depolymerization, thereby causing G2/M blockade of the cell cycle and destroying the mitotic spindle, ultimately leading to cell apoptosis. Taxanes and eribulin are both commonly used chemotherapy drugs in clinic. However, both drugs will develop a certain degree of drug resistance after a period of administration.

Therefore, there is still a need to find a way to improve the efficacy of single drugs in chemotherapy and further overcome the problem of drug resistance.

### SUMMARY

One aspect of the present disclosure provides use of an FAK inhibitor, a microtubule inhibitor, and an immune checkpoint inhibitor in the manufacture of a medicament for treating a tumor in a subject.

Yet another aspect of the present disclosure provides a pharmaceutical combination product of an FAK inhibitor, a microtubule inhibitor, and an immune checkpoint inhibitor for use in treating a tumor in a subject.

Yet another aspect of the present disclosure provides a method for treating a tumor, comprising administering a therapeutically effective amount of an FAK inhibitor, a microtubule inhibitor, and an immune checkpoint inhibitor to a subject in need thereof.

Yet another aspect of the present disclosure provides a kit or a pharmaceutically acceptable composition comprising: (a) an FAK inhibitor; (b) a microtubule inhibitor; and (c) an immune checkpoint inhibitor.

Yet another aspect of the present disclosure provides use of an FAK inhibitor and a microtubule inhibitor in the manufacture of a medicament for treating a tumor, wherein the FAK inhibitor is used to enhance the immunogenic cell death induced by the microtubule inhibitor.

Yet another aspect of the present disclosure provides an FAK inhibitor for use in enhancing immunogenic cell death induced by a microtubule inhibitor in the treatment of a tumor.

Yet another aspect of the present disclosure provides a method for treating a tumor, comprising administering a therapeutically effective amount of an FAK inhibitor and a microtubule inhibitor to a subject in need thereof, wherein the FAK inhibitor is used to enhance the immunogenic cell death induced by the microtubule inhibitor.

Yet another aspect of the present disclosure provides use of an FAK inhibitor, a microtubule inhibitor, and an immune checkpoint inhibitor in the manufacture of a medicament for combined treatment of a tumor.

Yet another aspect of the present disclosure provides use of an FAK inhibitor in the manufacture of a combined medicament with a microtubule inhibitor and an immune checkpoint inhibitor for treating a tumor.

Yet another aspect of the present disclosure provides use of a microtubule inhibitor in the manufacture of a combined medicament with an FAK inhibitor and an immune checkpoint inhibitor for treating a tumor.

Yet another aspect of the present disclosure provides use of an immune checkpoint inhibitor in the manufacture of a combined medicament with an FAK inhibitor and a microtubule inhibitor for treating a tumor.

Yet another aspect of the present disclosure provides use of an FAK inhibitor in the manufacture of a medicament for combined treatment of a tumor with a microtubule inhibitor and an immune checkpoint inhibitor.

Yet another aspect of the present disclosure provides use of a microtubule inhibitor in the manufacture of a medicament for combined treatment of a tumor with an FAK inhibitor and an immune checkpoint inhibitor.

Yet another aspect of the present disclosure provides use of an immune checkpoint inhibitor in the manufacture of a medicament for combined treatment of a tumor with an FAK inhibitor and a microtubule inhibitor.

Yet another aspect of the present disclosure provides a kit, which includes: an FAK inhibitor; and an instruction, wherein the instruction indicates that the FAK inhibitor can be used in combination with a microtubule inhibitor and an immune checkpoint inhibitor to treat a tumor.

Yet another aspect of the present disclosure provides a kit, which includes: a microtubule inhibitor; and an instruction, wherein the instruction indicates that the microtubule inhibitor can be used in combination with an FAK inhibitor and an immune checkpoint inhibitor to treat a tumor.

Yet another aspect of the present disclosure provides a kit, which includes: an immune checkpoint inhibitor; and an instruction, wherein the instruction indicates that the immune checkpoint inhibitor can be used in combination with an FAK inhibitor and a microtubule inhibitor to treat a tumor.

Yet another aspect of the present disclosure provides a method for treating a tumor, comprising administering a therapeutically effective amount of an FAK inhibitor and a microtubule inhibitor to a subject in need thereof.

Yet another aspect of the present disclosure provides a pharmaceutical combination product of an FAK inhibitor and a microtubule inhibitor for use in treating a tumor in a subject in need thereof.

Yet another aspect of the present disclosure provides use of an FAK inhibitor and a microtubule inhibitor in the manufacture of a combined medicament for treating a tumor.

Yet another aspect of the present disclosure provides use of an FAK inhibitor in the manufacture of a combined medicament with a microtubule inhibitor for treating a tumor.

Yet another aspect of the present disclosure provides use of a microtubule inhibitor in the manufacture of a combined medicament with an FAK inhibitor for treating a tumor.

Yet another aspect of the present disclosure provides use of an FAK inhibitor and a microtubule inhibitor in the manufacture of a medicament for combined treatment of a tumor.

Yet another aspect of the present disclosure provides use of an FAK inhibitor in the manufacture of a medicament for combined treatment of a tumor with a microtubule inhibitor.

Yet another aspect of the present disclosure provides use of a microtubule inhibitor in the manufacture of a medicament for combined treatment of a tumor with an FAK inhibitor.

Yet another aspect of the present disclosure provides a kit, which includes: an FAK inhibitor; and an instruction, wherein the instruction indicates that the FAK inhibitor can be used in combination with a microtubule inhibitor to treat a tumor.

Yet another aspect of the present disclosure provides a kit, which includes: a microtubule inhibitor; and an instruction, wherein the instruction indicates that the microtubule inhibitor can be used in combination with an FAK inhibitor to treat a tumor.

Optionally, the FAK inhibitor is IN10018, Defactinib, GSK2256098, PF-00562271, VS-4718, APG-2449, AMP945, AMP886 or a pharmaceutically acceptable salt thereof, alternatively IN10018, Defactinib, AMP945, or a pharmaceutically acceptable salt thereof, further alternatively IN10018 or a pharmaceutically acceptable salt thereof, alternatively IN10018 tartrate, and the structure of IN10018 is:

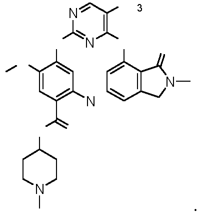

The Defactinib is also known as difatini, with a CAS number of 1345713-71-4; the CAS number of the GSK2256098 is 1224887-10-8. The CAS number of the PF-00562271 is 717907-75-0; the CAS number of the VS-4718 is 1061353-68-1; the APG-2449 is developed by Ascent Pharmaceuticals; the CAS number of the AMP945 is 1393653-34-3.

Optionally, the microtubule inhibitor is a taxane, Eribulin, Ixempra or a vinca alkaloid.

Optionally, the microtubule inhibitor is a taxane.

Optionally, the taxane is docetaxel, paclitaxel, cabazitaxel or cephalomannine, alternatively docetaxel or paclitaxel.

Optionally, the microtubule inhibitor is docetaxel.

Optionally, the microtubule inhibitor is paclitaxel.

The paclitaxel or docetaxel includes formulations thereof, such as paclitaxel liposome, albumin paclitaxel, and the like.

Optionally, the microtubule inhibitor is Eribulin, which is also called Erebulin/Halaven, and has a CAS number of 253128-41-5.

Optionally, the immune checkpoint inhibitor is an anti-PD-1/PD-L1 antibody, a PD-1/PD-L1 small molecule inhibitor or a TIGIT inhibitor.

Optionally, the immune checkpoint inhibitor is an anti-PD-1/PD-L1 antibody, and alternatively, the anti-PD-1/PD-L1 antibody is pembrolizumab, tislelizumab, nivolumab, toripalimab, atezolizumab, durvalumab, avelumab, camrelizumab, sintilimab, cemiplimab, envafolimab, BMS-936559, JS003, SHR-1316, GS-4224, AN-4005, or MX-10181.

Optionally, the immune checkpoint inhibitor is a PD-1/PD-L1 small molecule inhibitor, and alternatively, the PD-1/PD-L1 small molecule inhibitor is INCB-086550, Lazertinib, IMMH-010, CA-170, ABSK043 or RRx-001.

Optionally, the immune checkpoint inhibitor is a TIGIT inhibitor, and alternatively, the TIGIT inhibitor is Ociperlimab (BGB-A1217), Vibostolimab, domvanalimab (AB154), Tiragolumab, Belrestotug, Etigilimab, ONO-4686, JS-006, AZD-2936, HLX-301, SEA-TGT, M-6223, IBI-939, COM-902, AB-308, AGEN-1777, AK-127, BAT-6021, BAT-6005, ASP-8374, PM-1022, BMS-986207, HB0036, or IBI-321.

Optionally, the FAK inhibitor is IN10018 or a pharmaceutically acceptable salt thereof, the microtubule inhibitor is docetaxel; and the immune checkpoint inhibitor is an anti-PD-1/PD-L1 antibody or a PD-1/PD-L1 small molecule inhibitor.

Optionally, the FAK inhibitor is IN10018 or a pharmaceutically acceptable salt thereof, the microtubule inhibitor is docetaxel; and the immune checkpoint inhibitor is an anti-PD-1/PD-L1 antibody.

Optionally, the FAK inhibitor is IN10018 or a pharmaceutically acceptable salt thereof, the microtubule inhibitor is paclitaxel; and the immune checkpoint inhibitor is an anti-PD-1/PD-L1 antibody or a PD-1/PD-L1 small molecule inhibitor.

Optionally, the FAK inhibitor is IN10018 or a pharmaceutically acceptable salt thereof, the microtubule inhibitor is paclitaxel; and the immune checkpoint inhibitor is an anti-PD-1/PD-L1 antibody.

Optionally, the FAK inhibitor, the microtubule inhibitor and the immune checkpoint inhibitor are administered simultaneously or sequentially to the subject.

Optionally, the tumor is bladder cancer, breast cancer, cervical cancer, colon cancer (including colorectal cancer), esophageal cancer, esophageal squamous cell carcinoma, head and neck cancer, liver cancer, lung cancer (including small cell lung cancer and non-small cell lung cancer), melanoma, myeloma, rhabdomyosarcoma, inflammatory myofibroblastoma, neuroblastoma, pancreatic cancer, prostate cancer, kidney cancer, renal cell carcinoma, sarcoma (including osteosarcoma), skin cancer (including squamous cell carcinoma), gastric cancer, testicular cancer, thyroid cancer, uterine cancer, mesothelioma, cholangiocarcinoma, leiomyosarcoma, liposarcoma, nasopharyngeal carcinoma, neuroendocrine carcinoma, ovarian carcinoma, salivary adenocarcinoma, metastases caused by spindle cell carcinoma, anaplastic large cell lymphoma, undifferentiated thyroid cancer, non-Hodgkin's lymphoma, Hodgkin's lymphoma, glioma or hematologic malignancies such as acute myeloid leukemia (AML), acute lymphocytic leukemia (ALL), diffuse large B-cell lymphoma (DLBCL), follicular lymphoma (FL), chronic lymphocytic leukemia (CLL), or chronic myelogenous leukemia (CML).

Optionally, the tumor is breast cancer, ovarian cancer, colon cancer (including colorectal cancer), lung cancer (including small cell lung cancer and non-small cell lung cancer), melanoma, or pancreatic cancer.

Optionally, the tumor is breast cancer, ovarian cancer or colon cancer (including colorectal cancer).

### BRIEF DESCRIPTION OF DRAWINGS

In order to more clearly explain the technical solution of examples of the present disclosure, the drawings of examples will be briefly introduced below. Obviously, the drawings in the following description only relate to some examples of the present disclosure, and do not limit the present disclosure.

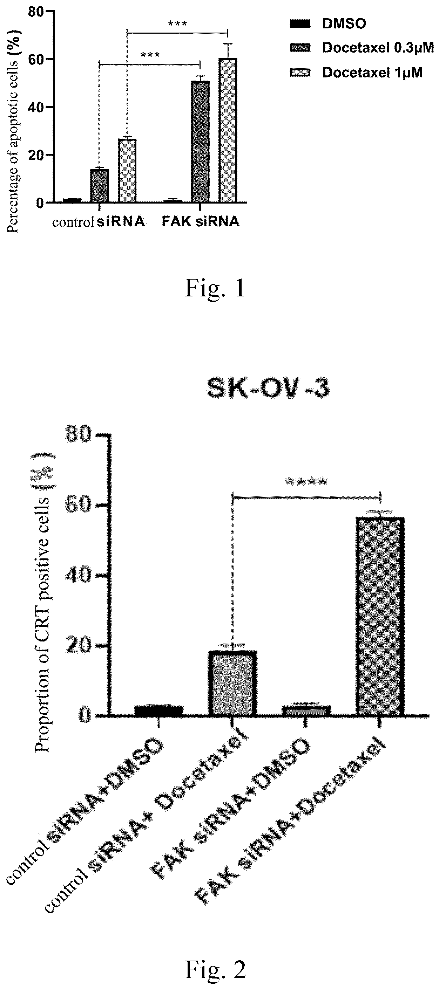

Fig. 1 shows the staining using the Annexin V kit after FAK silencing combined with 0.3 µM or 1 µM Docetaxel for 48 hours.
Fig. 2 shows that FAK silencing combined with 1 µM Docetaxel for 48 hours enhanced the release and exposure of calreticulin.

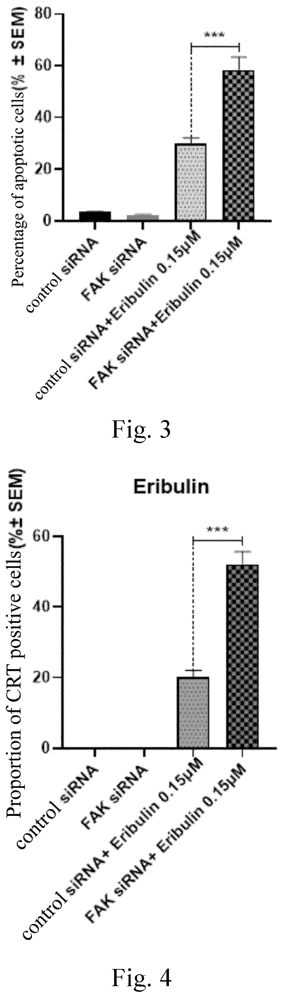

Fig. 3 shows the staining using the Annexin V kit after FAK silencing combined with 0.15 µM Eribulin for 48 hours.
Fig. 4 shows that FAK silencing combined with 0.15 µM Eribulin for 48 hours enhanced the release and exposure of calreticulin.

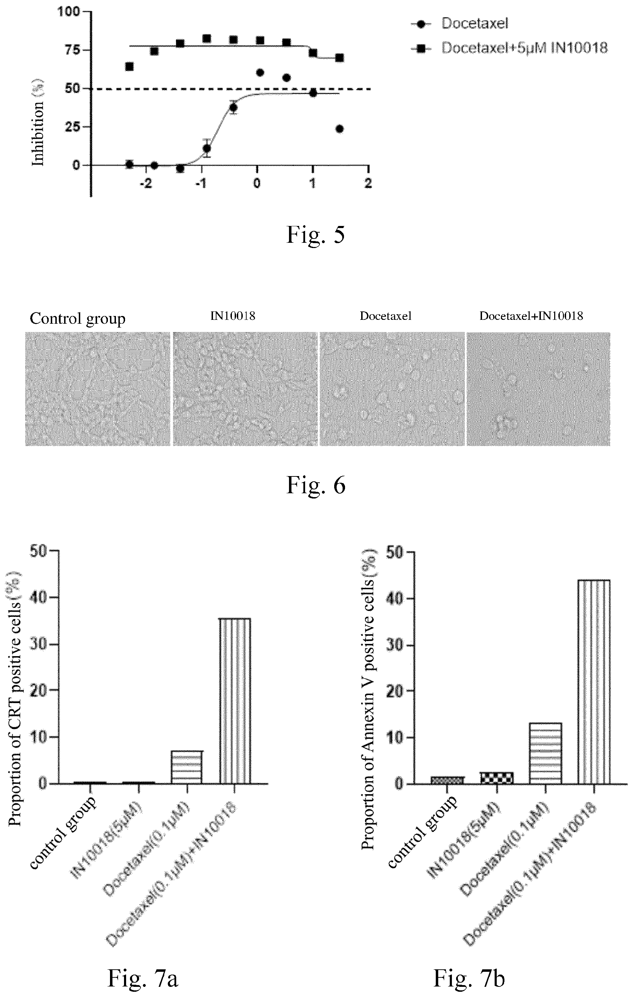

Fig. 5 shows the IC50 value of Docetaxel combined with IN10018 in colon cancer cells CT26.
Fig. 6 shows a white light microscope photograph of colon cancer CT26 cells after incubation with drugs for 48 hours.
Fig. 7a shows the percentage of CRT-positive cells after colon cancer CT26 cells were incubated with drugs for 48 hours; Fig. 7b shows the percentage of Annexin V-positive cells after colon cancer CT26 cells were incubated with drugs for 48 hours.

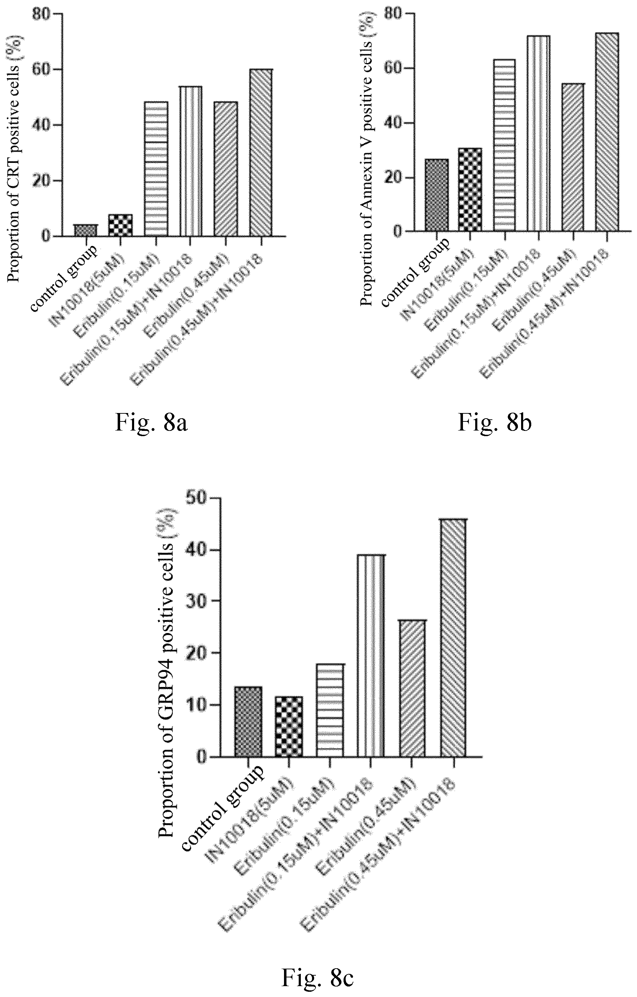

Fig. 8a shows the percentage of Calreticulin (CRT) positive cells after mouse breast cancer 4T1 cells were incubated with drugs for 48 hours; Fig. 8b shows the percentage of Annexin-V positive cells after mouse breast cancer 4T1 cells were incubated with drugs for 48 hours; Fig. 8c shows the percentage of GRP94 positive cells after mouse breast cancer 4T1 cells were incubated with drugs for 48 hours.

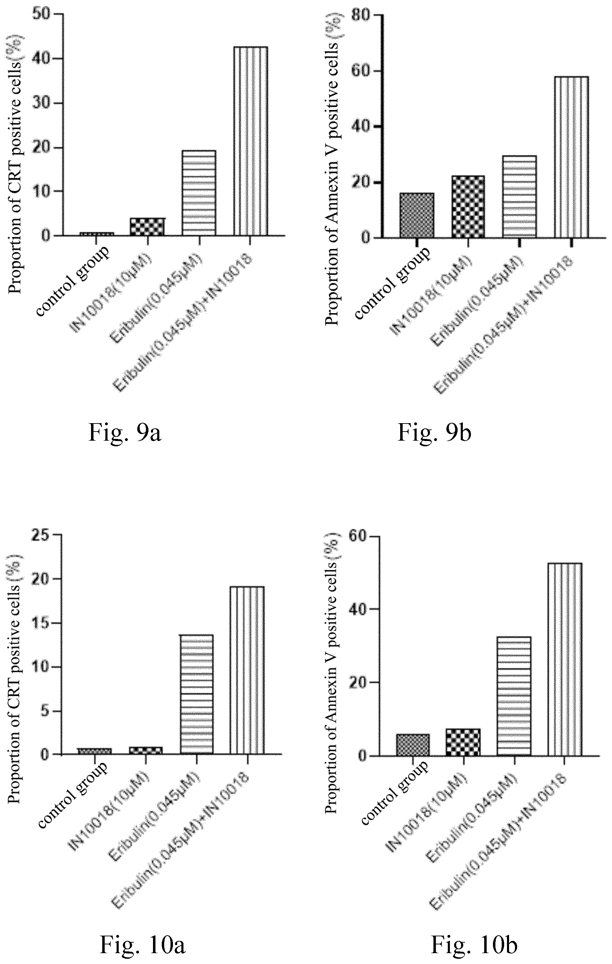

Fig. 9a shows the percentage of Calreticulin (CRT) positive cells after mouse ovarian epithelial carcinoma ID 8 cells were incubated with drugs for 48 hours; Fig. 9b shows the percentage of Annexin-V positive cells after mouse ovarian epithelial carcinoma ID 8 cells were incubated with drugs for 48 hours.
Fig. 10a shows the percentage of Calreticulin (CRT) positive cells after Human ovarian epithelial carcinoma TOV-21G cells were incubated with drugs for 48 hours; Fig. 10b shows the percentage of Annexin-V positive cells after Human ovarian epithelial carcinoma TOV-21G cells were incubated with drugs for 48 hours.

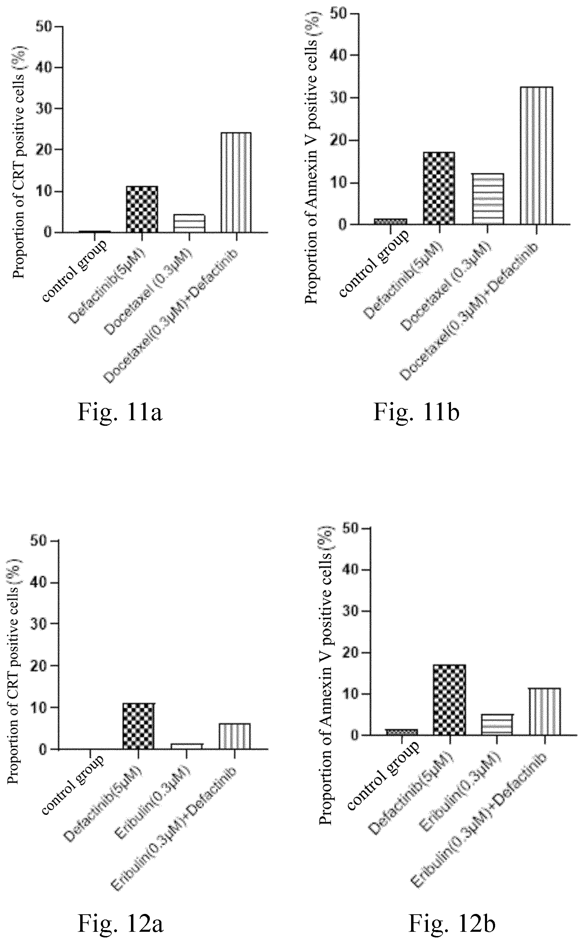

Fig. 11a shows the percentage of Calreticulin (CRT) positive cells after mouse colon cancer CT26 cells were incubated with Docetaxel and other drugs for 48 hours; Fig. 11b shows the percentage of Annexin-V positive cells after mouse colon cancer CT26 cells were incubated with Docetaxel and other drugs for 48 hours.
Fig. 12a shows the percentage of Calreticulin (CRT) positive cells after mouse colon cancer CT26 cells were incubated with Eribulin and other drugs for 48 hours; Fig. 12b shows the percentage of Annexin-V positive cells after mouse colon cancer CT26 cells were incubated with Eribulin and other drugs for 48 hours.

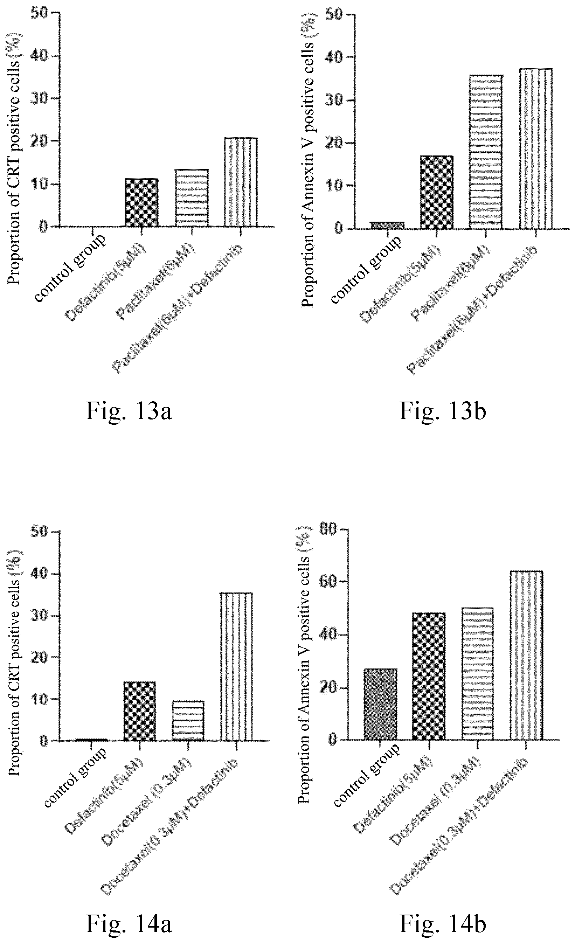

Fig. 13a shows the percentage of Calreticulin (CRT) positive cells after mouse colon cancer CT26 cells were incubated with Paclitaxel and other drugs for 48 hours; Fig. 13b shows the percentage of Annexin-V positive cells after mouse colon cancer CT26 cells were incubated with Paclitaxel and other drugs for 48 hours.
Fig. 14a shows the percentage of Calreticulin (CRT) positive cells after mouse breast cancer 4T1 cells were incubated with Docetaxel and other drugs for 48 hours; Fig. 14b shows the percentage of Annexin-V positive cells after mouse breast cancer 4T1 cells were incubated with Docetaxel and other drugs for 48 hours.

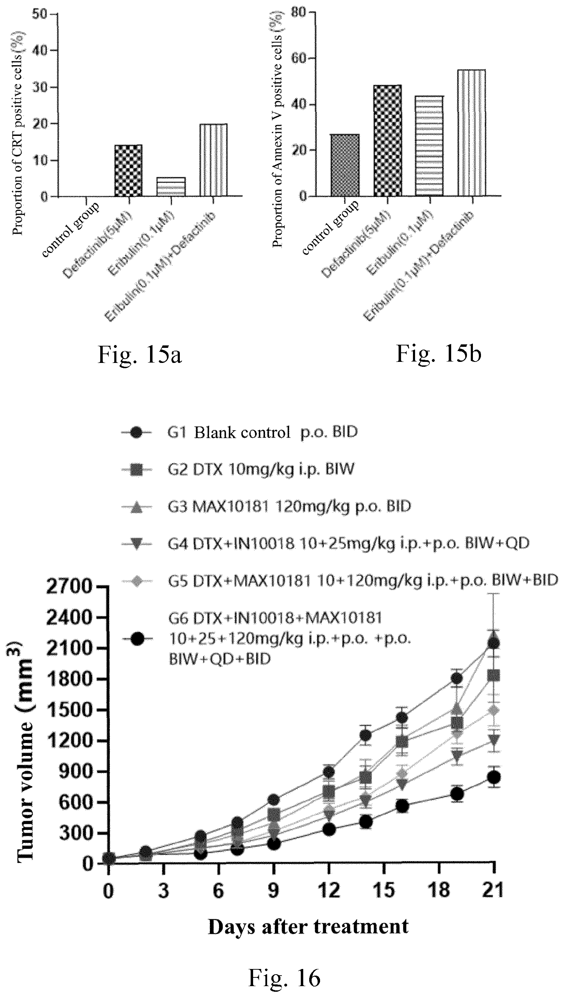

Fig. 15a shows the percentage of Calreticulin (CRT) positive cells after mouse breast cancer 4T1 cells were incubated with Eribulin and other drugs for 48 hours; Fig. 15b shows the percentage of Annexin-V positive cells after mouse breast cancer 4T1 cells were incubated with Eribulin and other drugs for 48 hours.
Fig. 16 shows the changes in tumor volume after administration of different test substances in a subcutaneous BALB/c mouse allograft tumor model of breast cancer 4T1 cells.

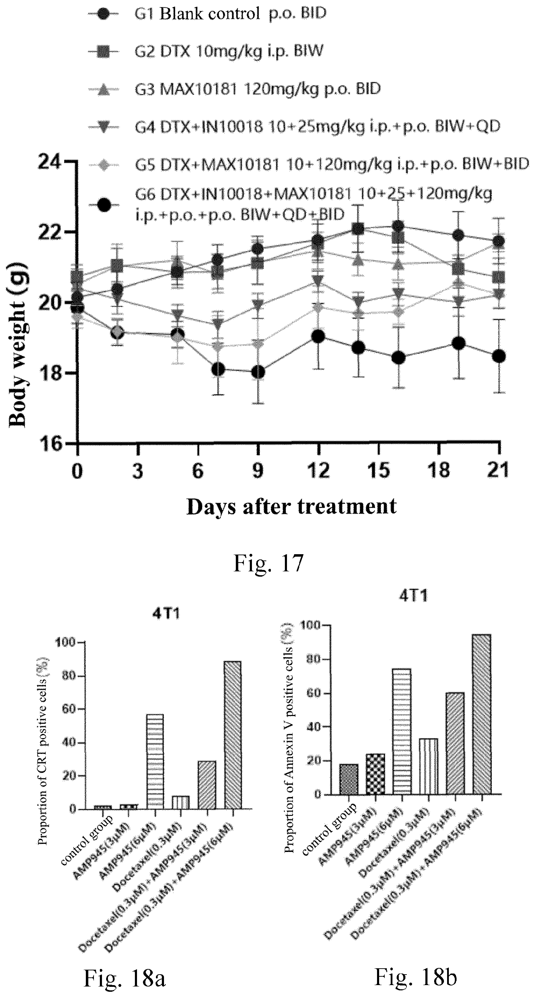

Fig. 17 shows the changes in body weight of mice after administration of different test substances in a subcutaneous BALB/c mouse allograft tumor model of breast cancer 4T1 cells.
Fig. 18a shows the percentage of Calreticulin (CRT) positive cells after mouse breast cancer 4T1 cells were incubated with Docetaxel and other drugs for 48 hours; Fig. 18b shows the percentage of Annexin-V positive cells after mouse breast cancer 4T1 cells were incubated with Docetaxel and other drugs for 48 hours.

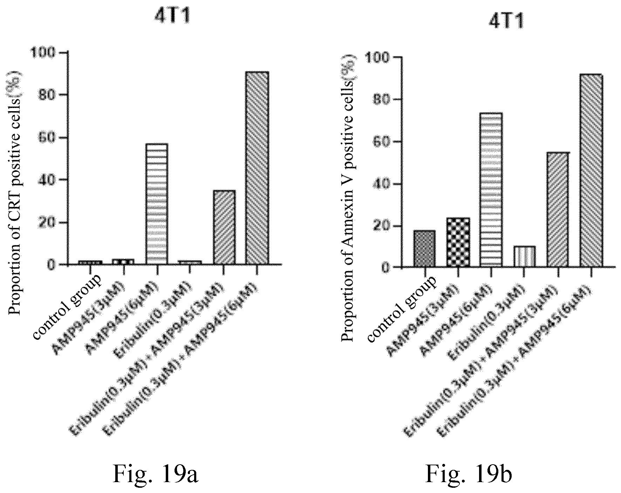

Fig. 19a shows the percentage of Calreticulin (CRT) positive cells after mouse breast cancer 4T1 cells were incubated with Eribulin and other drugs for 48 hours; Fig. 19b shows the percentage of Annexin-V positive cells after mouse breast cancer 4T1 cells were incubated with Eribulin and other drugs for 48 hours.

### DETAILED DESCRIPTION

In order to make the purpose, technical solutions and advantages of the examples of the present disclosure more clear, the technical solution of the example of the present disclosure will be described clearly and completely with the attached Figs. Obviously, the described example is a part of examples of the present disclosure, not the whole examples. Based on the described examples of the present disclosure, all other examples obtained by ordinary people in the field without creative labor belong to the scope of protection of the present disclosure.

The present disclosure can be embodied in other specific forms without departing from the basic attributes of the present disclosure. It should be understood that any and all embodiments of the present disclosure can be combined with the technical features of any other embodiment or a plurality of other embodiments to obtain additional embodiments without conflict. The present disclosure includes further embodiments resulting from such combinations.

All publications and patents mentioned in this disclosure are hereby incorporated by reference in their entirety. If the use or terminology used in any publication and patent incorporated by reference conflicts with the use or terminology used in this disclosure, the use and terminology of the present disclosure shall prevail.

The chapter headings used in this disclosure are only for the purpose of organizing articles and should not be interpreted as limitations on the subject.

Unless otherwise specified, all technical and scientific terms used herein have the usual meaning in the field to which the claimed subject matter belongs. If there are multiple definitions of a term, the definition in the present disclosure shall prevail.

Except in working examples or otherwise indicated, all numbers of quantitative properties such as dosage stated in the description and claims should be understood as being modified in all instances by the term "about". It should also be understood that any numerical range recited in this application is intended to include all subranges within the range and any combination of endpoints of the range or subrange.

"Including", "containing", "comprising" or the like used in this disclosure means that the elements appearing before the word cover the elements listed after the word and their equivalents, but do not exclude elements that are not recorded. The term "including", "containing", or "comprising" used herein can be open, semi-closed and closed. In other words, the term also includes "consisting essentially of" or "consisting of".

### Definition

The following terms and symbols used in this application have the following meanings, unless otherwise specified in the context. As used herein, the term "FAK inhibitor" refers to an effective inhibitor of FAK, which can be suitable for mammals, especially humans. In some embodiments, the FAK inhibitor is IN10018, Defactinib, GSK2256098, PF-00562271, VS-4718, APG-2449, AMP945, AMP886 or pharmaceutically acceptable salts thereof, wherein the structure of IN10018 is 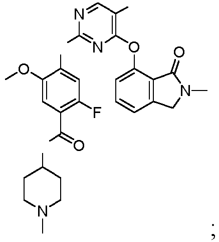 the Defactinib is also known as difatini, and the CAS number is 1345713-71-4; the CAS number of GSK2256098 is 1224887-10-8; the CAS number of PF-00562271 is 717907-75-0; the CAS number of VS-4718 is 1061353-68-1; the APG-2449 is developed by Yasheng Medicine; the CAS number of AMP945 is 1393653-34-3. In some embodiments, the FAK inhibitor is alternatively IN10018, Defactinib, or a pharmaceutically acceptable salt thereof, and in some alternative embodiments, the FAK inhibitor is IN10018 or a pharmaceutically acceptable salt thereof, especially IN10018 tartrate.

As used herein, the term "microtubule inhibitor" refers to an effective inhibitor of microtubules/tubulin, which can be suitable for mammals, especially humans. Microtubules are part of cytoskeleton and are distributed throughout the cytoplasm. Tubulin is one of several members of a small family of globulins. The tubulin superfamily includes 5 different families, α, β, γ, δ and ε tubulin, and the sixth family is found only in embryonic protozoa of plants and animals. The most common members of the tubulin family are α-tubulin and β-tubulin, which are proteins that constitute microtubules. Microtubule inhibitors bind to tubulin to promote or inhibit the assembly of microtubules, to interfere with the normal structure and function of the spindle composed of microtubules, to have the effect of interfering with cell mitosis and inhibiting cell proliferation, and to exert a clear anti-tumor effect. Microtubule inhibitors are a large class of chemotherapeutic drugs, including taxanes, eribulin, ixabepilone and vinca alkaloids, etc.

The term "taxanes" used herein refers to a large class of chemotherapy drugs, including paclitaxel extracted from natural bark, and synthetic docetaxel. To avoid ambiguity, paclitaxel or docetaxel in the claims of the present disclosure include their formulations, such as paclitaxel liposome, albumin paclitaxel, etc.

The eribulin is also called Erebulin/Halaven, and its CAS number is 253128-41-5.

The ixabepilone is also known as Ixabepilone, azaepothilone B or BMS-247550, and its CAS number is 219989-84-1.

As used herein, the term "immune checkpoint inhibitor" refers to a medicament that can improve the immune system activity by regulating immune checkpoint pathways (such as PD-1, TIGIT, CTLA-4, LAG-3, TIM-3, etc.). In some embodiments, the immune checkpoint inhibitor is an antagonist of the PD-1/PD-L1 (programmed cell death protein 1) pathway (also called "PD-1 inhibitor") or a TIGIT inhibitor. PD-1 inhibitors are also referred to as PD-1/PD-L1 inhibitors in this disclosure. For example, in the therapeutic methods, medicaments and uses of the present disclosure, the PD-1/PD-L1 inhibitor is a PD-1/PD-L1 antibody, including, but not limited to, pabolizumab (KERIDA/Keytruda/K medicament), Tislelizumab (Baizean), Nivolumab, Toripalimab (Tuoyi), durvalumab (Imfinzi), Avelumab (Bavencio), Atezolizumab (MPDL 3280A/Tecentriq/T medicament), BMS-936559 (fully humanized IgG4 monoclonal antibody against PD-L1), GS-4224, AN-4005 or MX-10181. In some alternative embodiments, the PD-1 inhibitor is Toripalimab. In some embodiments, PD-1 inhibitors are used to treat human subjects. In some embodiments, PD-1 is human PD-1.

PD-1/PD-L1 inhibitors also include PD-1/PD-L1 small molecule inhibitors. In some embodiments, the PD-1/PD-L1 small molecule inhibitors are INCB-086550, Lazertinib, IMMH-010, CA-170, ABSK043 or RRx-001.

TIGIT (also known as WUCAM, Vstm3, VSIG9) is a receptor of the Ig superfamily and is a new immune checkpoint besides PD-1/PD-L1. For example, in the therapeutic methods, medicaments and uses of the present disclosure, the PD-1/PD-L1 inhibitor is a TIGIT inhibitor, including, but not limited to, Ociperlimab (BGB-A1217), Vibostolimab, domvanalimab (AB154), Tiragolumab, Belrestotug, Etigilimab, ONO-4686, JS-006, AZD-2936, HLX-301, SEA-TGT, M-6223, IBI-939, COM-902, AB-308, AGEN-1777, AK-127, BAT-6021, BAT-6005, ASP-8374, PM-1022, BMS-986207, HB0036, or IBI-321. In some embodiments, the TIGIT inhibitors are used to treat human subjects. In order to avoid ambiguity, all antibodies in the present disclosure include double antibodies.

As used herein, "pharmaceutical combination" or "pharmaceutical combination product" can refer to the case of a fixed combination in the form of a dosage unit (for example, all pharmaceutical active ingredients exist in one dosage form) or the case of a product that is administered in combination in a complete kit, and the case of a combination of a medicament and instructions indicating that the medicament can be used in combination with one or more other medicaments.

As used herein, "combined treatment" or "combined medicament" means that a medicament is used in combination with one or more other medicaments to treat a disease, including both the combination of one medicament and one or more other medicaments and the combination of one medicament and instructions indicating that the medicament can be used in combination with one or more other medicaments.

In this application, "simultaneous or sequential administration" refers to the simultaneous administration of two or more medicaments or sequential administration of two or more medicaments at a certain time interval within an administration cycle (for example, within 4 weeks, 3 weeks, 2 weeks, 1 week or 24 hours). The modes of administration (for example, oral administration, intravenous administration, intramuscular administration or subcutaneous administration) may be the same or different, and the frequency/cycle of administration of two or more medicaments may be the same or different. When the therapeutic method, product or use of the present disclosure involves two medicaments, the two medicaments can be administered simultaneously or separately at a certain interval. When the therapeutic method, product or use of the present disclosure involves three medicaments, the three medicaments can be administered at the same time point, or two medicaments can be administered at one time point and the remaining one medicament can be administered at another time point, or each of the three medicaments can be administered at different time points.

In some embodiments, the PD-1/PD-L1 inhibitor is administered intravenously (e.g., as intravenous infusion), subcutaneously or orally. Alternatively, the PD-1/PD-L1 inhibitor is administered by intravenous infusion.

In some embodiments, the TIGIT inhibitor is administered intravenously (e.g., as intravenous infusion), subcutaneously or orally. Alternatively, the TIGIT inhibitor is administered by intravenous infusion.

The ability of immune checkpoint inhibitors to treat cancer depends on the existence of tumor antigen-specific T cells in a tumor tissue. This requires tumor tissues to express antigens that distinguish themselves from their untransformed counterparts, for example, through a new protein product called neoantigen. The neoantigen load of tumor is closely related to immunogenicity and sensitivity (for example, sensitivity to checkpoint inhibitor therapy), which means that tumors with poor immunogenicity should be resistant to these medicaments to a great extent. Therapeutics for releasing tumor antigens that can be ingested by APC, such as those that induce immunogenic cell death (ICD), may promote effective anti-tumor immunity, especially when further combined with a checkpoint inhibitor.

As used herein, the term "treatment" refers to the administration of one or more pharmaceutical substances to a subject suffering from a disease or having symptoms of the disease in order to cure, alleviate, mitigate, change, treat, improve, ameliorate or influence the disease or symptoms of the disease. In some embodiments, the disease is a tumor or cancer.

As used herein, the term "tumor" refers to the abnormal pathological changes formed by the abnormal proliferation of clonal cells caused by the loss of normal regulation of the growth of cells of local tissues at the gene level under the action of various tumorigenic factors. The tumor includes, but is not limited to: bladder cancer, breast cancer, cervical cancer, colon cancer (including colorectal cancer), esophageal cancer, esophageal squamous cell carcinoma, head and neck cancer, liver cancer, lung cancer (including small cell lung cancer and non-small cell lung cancer), melanoma, myeloma, rhabdomyosarcoma, inflammatory myofibroblastoma, neuroblastoma, pancreatic cancer, prostate cancer, kidney cancer, renal cell carcinoma, sarcoma (including osteosarcoma), skin cancer (including squamous cell carcinoma), gastric cancer, testicular cancer, thyroid cancer, uterine cancer, mesothelioma, cholangiocarcinoma, leiomyosarcoma, liposarcoma, nasopharyngeal carcinoma, neuroendocrine carcinoma, ovarian carcinoma, salivary adenocarcinoma, metastases caused by spindle cell carcinoma, anaplastic large cell lymphoma, undifferentiated thyroid cancer, non-Hodgkin's lymphoma, Hodgkin's lymphoma, glioma or hematologic malignancies such as acute myeloid leukemia (AML), acute lymphocytic leukemia (ALL), diffuse large B-cell lymphoma (DLBCL), follicular lymphoma (FL), chronic lymphocytic leukemia (CLL), or chronic myelogenous leukemia (CML). In some embodiments, the tumor is alternatively breast cancer, ovarian cancer, colon cancer (including colorectal cancer), lung cancer (including small cell lung cancer and non-small cell lung cancer), melanoma or pancreatic cancer; In some embodiments, the tumor is breast cancer, ovarian cancer, or colon cancer (including colorectal cancer).

As used herein, the term "subject" or "patient" refers to mammals and non-mammals. Mammals refer to any member of mammals, including but not limited to: human; non-human primates, such as chimpanzees and other apes and monkey species; farm animals such as cattle, horses, sheep, goats and pigs; domestic animals such as rabbits, dogs and cats; laboratory animals, including rodents, such as rats, mice and guinea pigs, etc. Examples of non-mammals include but are not limited to birds, etc. The term "subject" is not limited to a specific age or gender. In some embodiments, the subject is a human.

As used herein, the term "pharmaceutically acceptable" means non-toxic, biologically tolerable and suitable for administration to a subject.

As used herein, the term "pharmaceutically acceptable salt" refers to non-toxic and biologically tolerable acid addition salts suitable for administration to subjects, including but not limited to: acid addition salts formed with inorganic acids, such as hydrochloride, hydrobromide, carbonate, bicarbonate, phosphate, sulfate, sulfite, nitrate, etc.; and acid addition salts with organic acids, such as formate, acetate, malate, maleate, fumarate, tartrate, succinate, citrate, lactate, methanesulfonate, p-toluenesulfonate, 2-hydroxyethyl sulfonate, benzoate, salicylate, stearate and salts formed with alkanedicarboxylic acids of formula HOOC-(CH₂)ₙ-COOH (where n is 0-4), etc.

Furthermore, a pharmaceutically acceptable acid addition salt can be obtained by dissolving a free base in a suitable solvent and treating the solution with an acid according to a conventional operation for preparing an acid addition salt from a basic compound. Those skilled in the art can determine various synthetic methods that can be used to prepare nontoxic pharmaceutically acceptable acid addition salts without redundant experiments.

As used herein, the term "pharmaceutically acceptable composition" means that it must be chemically and/or toxicologically compatible with other ingredients included in the preparation, and/or compatible with the subject to be treated. As used herein, the term "therapeutically effective amount" refers to an amount generally sufficient to produce a beneficial therapeutic effect on a subject. The therapeutically effective amount of the present disclosure can be determined by conventional methods (such as modeling, dose escalation study or clinical trial) combined with conventional influencing factors (such as administration mode, pharmacokinetics of compounds, severity and course of diseases, medical history of subjects, health status of subjects, response degree of subjects to medicaments, etc.).

As used herein, the term "inhibition" refers to a decrease in the baseline activity of a biological activity or process.

As used herein, the term "kit" refers to a box for containing chemical reagents for detecting chemical components, medicament residues, virus types and the like. The kit of the present disclosure can include (i) one, two or three of an FAK inhibitor, a microtubule inhibitor and an immune checkpoint inhibitor; and (ii) an instruction indicating that the FAK inhibitor, the microtubule inhibitor and the immune checkpoint inhibitor can be used to treat a tumor in the subject. In one embodiment, the kit includes (i) an FAK inhibitor; and (ii) an instruction indicating that the FAK inhibitor, a microtubule inhibitor and an immune checkpoint inhibitor can be used to treat a tumor in a subject. In one embodiment, the kit includes (i) a microtubule inhibitor; and (ii) an instruction indicating that an FAK inhibitor, the microtubule inhibitor and an immune checkpoint inhibitor can be used to treat a tumor in a subject. In one embodiment, the kit includes (i) an immune checkpoint inhibitor; and (ii) an instruction indicating that an FAK inhibitor, a microtubule inhibitor and the immune checkpoint inhibitor can be used to treat a tumor in a subject. In one embodiment, the kit includes (i) an FAK inhibitor, a microtubule inhibitor and an immune checkpoint inhibitor; and (ii) an instruction indicating that the FAK inhibitor, the microtubule inhibitor and the immune checkpoint inhibitor can be used to treat a tumor in a subject. In one embodiment, the kit includes (i) an FAK inhibitor; and (ii) an instruction indicating that the FAK inhibitor and a microtubule inhibitor can be used to treat a tumor in a subject. In one embodiment, the kit includes (i) a microtubule inhibitor; and (ii) an instruction indicating that the microtubule inhibitor and an FAK inhibitor can be used to treat a tumor in a subject.

The compounds of a kit can be contained in separate containers. Optionally, two or more compounds are contained in the same container. For example, the kit may include a first container, a second container, a third container, and a package insert, wherein the first container includes at least one dose of a medicament including an FAK inhibitor, the second container includes at least one dose of a microtubule inhibitor, and the third container includes at least one dose of a medicament including an immune checkpoint inhibitor, and the package insert includes instructions of treating a tumor in a subject with the medicament. The first container, the second container and the third container may contain the same or different shapes (e.g., vials, syringes and bottles) and/or materials (e.g., plastic or glass). The kit can also include other materials that can help to administer medicaments, such as diluents, filters, IV bags and lines, needles and syringes.

The exact amount of the FAK inhibitor, the microtubule inhibitor and the immune checkpoint inhibitor to be administered to a subject will depend on various factors, such as a given medicament or compound, pharmaceutical preparation, route of administration, type of disease, symptoms, identity of the subject or host to be treated, etc., but it can still be routinely determined by those skilled in the art. For example, determining the effective amount also depends on the degree, severity and type of cell proliferation. The skilled person will be able to determine the appropriate dosage based on these and other factors.

The FAK inhibitor, the microtubule inhibitor and the immune checkpoint inhibitor can be administered in an appropriate manner, such as oral administration, intravenous administration, intramuscular administration, or subcutaneous administration.

For example, when administered orally, the medicament can be administered orally together with a pharmaceutically acceptable carrier such as an inert diluent or an absorbable edible carrier. They can be encapsulated in hard-shell or soft-shell gelatin capsules, can be pressed into tablets, or can be directly mixed with patients' food. For example, medicaments can be combined with one or more excipients and used in the form of ingestible tablets, oral tablets, lozenges, capsules, elixirs, suspensions, syrups or wafers. Tablets, lozenges, pills, capsules, and the like may further include a binder, such as tragacanth gum, arabic gum, corn starch or gelatin; an excipient, such as dicalcium phosphate; a disintegrator, such as corn starch, potato starch, alginic acid, and the like; a lubricant, such as magnesium stearate; or a sweetener, such as sucrose, fructose, lactose or aspartame; or a flavoring agent.

For example, when administered intravenously or intraperitoneally by infusion or injection, the medicament solution can be prepared in water, optionally mixed with a non-toxic surfactant.

Exemplary pharmaceutical dosage forms for injection or infusion include a sterile aqueous solution, a dispersion, or a sterile powder containing an active ingredient, which is suitable for temporarily preparing a sterile injection or infusion solution or dispersion. In any case, the final dosage form should be sterile, fluid and stable under the conditions of production and storage.

Sterile injection solution can be prepared by mixing the required amount of medicaments with various other components mentioned above into a suitable solvent, and then filtering and sterilizing the mixture. For sterile powder used to prepare sterile injection solution, the alternative preparation methods can be vacuum drying and freeze drying techniques, which can produce powder of active ingredient plus any other required ingredients existing after previous sterile filtration.

The amount of the FAK inhibitor, the microtubule inhibitor and the immune checkpoint inhibitor required for treatment can vary not only with the specific reagents selected, but also with the route of administration, the nature of the diseases to be treated, and the age and condition of the patients, and can be finally decided by the attending physician or clinician. However, in general, the dosage can be in the range of about 0.1 to about 50mg/kg body weight per day.

The FAK inhibitor is administered in a dosage range of 5mg/day to 300 mg/day in adults. In a specific embodiment, IN10018 or a pharmaceutically acceptable salt thereof is administered at a dose of 5 mg/day to 100 mg/day in adults. In a specific embodiment, IN10018 or a pharmaceutically acceptable salt thereof is administered at a dose of 25 mg/day to 100 mg/day in adults, and the dose is calculated as free base.

The microtubule inhibitor is administered in a dose of 20-60 mg/m² per week in adults. In a specific embodiment, docetaxel is administered in a dose of 20-25 mg/m² per week in adults; paclitaxel or its formulation is administered in a dose of 45-60 mg/m² per week in adults, calculated as paclitaxel; Eribulin is administered at 1.4 mg/m² intravenously over 2 to 5 minutes on the 1st and 8th days of a 21-day course of treatment.

The immune checkpoint inhibitor is administered each time at a dose of 2-10mg/kg or 50-1200mg for adults, and once every 2 to 3 weeks. In a specific embodiment, the immune checkpoint inhibitor is administered each time at a dose of 3-10mg/kg or 100-1200mg for adults and once every two to three weeks.

Technical and scientific terms not specifically defined used herein have the meanings commonly understood by those skilled in the art to which this disclosure belongs.

In some embodiments, the present disclosure also discloses:
1. An FAK inhibitor, a microtubule inhibitor and an immune checkpoint inhibitor, for use in a method for treating a tumor in a subject.
2. The FAK inhibitor, the microtubule inhibitor and the immune checkpoint inhibitor according to embodiment 1, wherein the FAK inhibitor is IN10018, Defactinib, GSK2256098, PF-00562271, VS-4718, APG-2449, AMP945, AMP886 or a pharmaceutically acceptable salt thereof, alternatively IN10018, Defactinib, AMP945 or a pharmaceutically acceptable salt thereof, yet alternatively IN10018 or a pharmaceutically acceptable salt thereof, alternatively IN10018 tartrate, and the structure of IN10018 is: 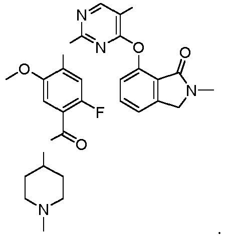
3. The FAK inhibitor, the microtubule inhibitor and the immune checkpoint inhibitor according to embodiment 1 or 2, wherein the microtubule inhibitor is a taxane, Eribulin, Ixempra or vinca alkaloid.
4. The FAK inhibitor, the microtubule inhibitor and the immune checkpoint inhibitor according to any one of embodiments 1-3, wherein the microtubule inhibitor is a taxane.
5. The FAK inhibitor, the microtubule inhibitor and the immune checkpoint inhibitor according to any one of embodiments 3-4, wherein the taxane is Docetaxel, Paclitaxel, Cabazitaxel or Cephalomannine, alternatively Docetaxel or Paclitaxel.
6. The FAK inhibitor, the microtubule inhibitor and the immune checkpoint inhibitor according to any one of embodiments 1-5, wherein the microtubule inhibitor is Docetaxel.
7. The FAK inhibitor, the microtubule inhibitor and the immune checkpoint inhibitor according to any one of embodiments 1-5, wherein the microtubule inhibitor is Paclitaxel.
8. The FAK inhibitor, the microtubule inhibitor and the immune checkpoint inhibitor according to any one of embodiments 1-4, wherein the microtubule inhibitor is Eribulin.
9. The FAK inhibitor, the microtubule inhibitor and the immune checkpoint inhibitor according to any one of embodiments 1 to 8, wherein the immune checkpoint inhibitor is an anti-PD-1/PD-L1 antibody, a PD-1/PD-L1 small molecule inhibitor or a TIGIT inhibitor.
10. The FAK inhibitor, the microtubule inhibitor and the immune checkpoint inhibitor according to any one of embodiments 1 to 9, wherein the immune checkpoint inhibitor is an anti-PD-1/PD-L1 antibody, and optionally the anti-PD-1/PD-L1 antibody is Pembrolizumab, Tislelizumab, Nivolumab, Toripalimab, Atezolizumab, Durvalumab, Avelumab, Camrelizumab, Sintilimab, Cemiplimab, Envafolimab, BMS-936559, JS003, SHR-1316, GS-4224, AN-4005, or MX-10181.
11. The FAK inhibitor, the microtubule inhibitor and the immune checkpoint inhibitor according to any one of embodiments 1-9, wherein the immune checkpoint inhibitor is a PD-1/PD-L1 small molecule inhibitor, and optionally the PD-1/PD-L1 small molecule inhibitor is INCB-086550, Lazertinib, IMMH-010, CA-170, ABSK043 or RRx-001.
12. The FAK inhibitor, the microtubule inhibitor and the immune checkpoint inhibitor according to any one of embodiments 1 to 9, wherein the immune checkpoint inhibitor is a TIGIT inhibitor, and optionally, the TIGIT inhibitor is Ociperlimab (BGB-A1217), Vibostolimab, Domvanalimab (AB154), Tiragolumab, Belrestotug, Etigilimab, ONO-4686, JS-006, AZD-2936, HLX-301, SEA-TGT, M-6223, IBI-939, COM-902, AB-308, AGEN-1777, AK-127, BAT-6021, BAT-6005, ASP-8374, PM-1022, BMS-986207, HB0036, or IBI-321.
13. The FAK inhibitor, the microtubule inhibitor and the immune checkpoint inhibitor according to embodiment 1, wherein the FAK inhibitor is IN10018 or a pharmaceutically acceptable salt thereof, the microtubule inhibitor is Docetaxel, and the immune checkpoint inhibitor is an anti-PD-1/PD-L1 antibody or a PD-1/PD-L1 small molecule inhibitor, and alternatively, the FAK inhibitor is IN10018 or a pharmaceutically acceptable salt thereof, the microtubule inhibitor is Docetaxel; the immune checkpoint inhibitor is an anti-PD-1/PD-L1 antibody.
14. The FAK inhibitor, the microtubule inhibitor and the immune checkpoint inhibitor according to embodiment 1, wherein the FAK inhibitor is IN10018 or a pharmaceutically acceptable salt thereof, the microtubule inhibitor is Paclitaxel, and the immune checkpoint inhibitor is an anti-PD-1/PD-L1 antibody or a PD-1/PD-L1 small molecule inhibitor, and alternatively, the FAK inhibitor is IN10018 or a pharmaceutically acceptable salt thereof, the microtubule inhibitor is Paclitaxel; the immune checkpoint inhibitor is an anti-PD-1/PD-L1 antibody.
15. The FAK inhibitor, the microtubule inhibitor and the immune checkpoint inhibitor according to any one of embodiments 1 to 14, wherein the FAK inhibitor, the microtubule inhibitor and the immune checkpoint inhibitor are administered simultaneously or sequentially to the subject.
16. The FAK inhibitor, the microtubule inhibitor and the immune checkpoint inhibitor according to any one of embodiments 1 to 15, wherein the tumor is bladder cancer, breast cancer, cervical cancer, colon cancer (including colorectal cancer), esophageal cancer, esophageal squamous cell carcinoma, head and neck cancer, liver cancer, lung cancer (including small cell lung cancer and non-small cell lung cancer), melanoma, myeloma, rhabdomyosarcoma, inflammatory myofibroblastoma, neuroblastoma, pancreatic cancer, prostate cancer, kidney cancer, renal cell carcinoma, sarcoma (including osteosarcoma), skin cancer (including squamous cell carcinoma), gastric cancer, testicular cancer, thyroid cancer, uterine cancer, mesothelioma, cholangiocarcinoma, leiomyosarcoma, liposarcoma, nasopharyngeal carcinoma, neuroendocrine carcinoma, ovarian carcinoma, salivary adenocarcinoma, metastases caused by spindle cell carcinoma, anaplastic large cell lymphoma, undifferentiated thyroid cancer, non-Hodgkin's lymphoma, Hodgkin's lymphoma, glioma or hematologic malignancies such as acute myeloid leukemia (AML), acute lymphocytic leukemia (ALL), diffuse large B-cell lymphoma (DLBCL), follicular lymphoma (FL), chronic lymphocytic leukemia (CLL), or chronic myelogenous leukemia (CML); alternatively, the tumor is breast cancer, ovarian cancer, colon cancer (including colorectal cancer), lung cancer (including small cell lung cancer and non-small cell lung cancer), melanoma, or pancreatic cancer.
17. The FAK inhibitor, the microtubule inhibitor and the immune checkpoint inhibitor according to any one of embodiments 1 to 16, wherein the tumor is breast cancer, ovarian cancer, or colon cancer (including colorectal cancer).
18. A kit or a pharmaceutically acceptable composition, comprising:
   (a) an FAK inhibitor;
   (b) a microtubule inhibitor; and
   (c) an immune checkpoint inhibitor.
19. The kit or composition according to embodiment 18, wherein the FAK inhibitor is IN10018, Defactinib, GSK2256098, PF-00562271, VS-4718, APG-2449, AMP945, AMP886, or a pharmaceutically acceptable salt thereof, alternatively, the FAK inhibitor is IN10018, Defactinib, AMP945 or a pharmaceutically acceptable salt thereof, and yet alternatively, the FAK inhibitor is IN10018, or a pharmaceutically acceptable salt thereof, alternatively, the FAK inhibitor is IN10018 tartrate, wherein the IN10018 has a structure of: 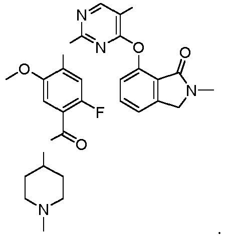
20. The kit or composition according to any one of embodiments 18-19, wherein the microtubule inhibitor is a taxane, Eribulin, Ixempra or a vinca alkaloid.
21. The kit or composition according to any one of embodiments 18-20, wherein the microtubule inhibitor is a taxane.
22. The kit or composition according to any one of embodiments 20-21, wherein the taxane is Docetaxel, Paclitaxel, Cabazitaxel or Cephalomannine, alternatively Docetaxel or Paclitaxel.
23. The kit or composition according to any one of embodiments 18-22, wherein the microtubule inhibitor is Docetaxel.
24. The kit or composition according to any one of embodiments 18-22, wherein the microtubule inhibitor is Paclitaxel.
25. The kit or composition according to any one of embodiments 18-22, wherein the microtubule inhibitor is Eribulin.
26. The kit or composition according to any one of embodiments 18-25, wherein the immune checkpoint inhibitor is an anti-PD-1/PD-L1 antibody, a PD-1/PD-L1 small molecule inhibitor, or a TIGIT inhibitor.
27. The kit or composition according to any one of embodiments 18-26, wherein the immune checkpoint inhibitor is an anti-PD-1/PD-L1 antibody, optionally, the anti-PD-1/PD-L1 antibody is Pembrolizumab, Tislelizumab, Nivolumab, Toripalimab, Atezolizumab, Durvalumab, Avelumab, Camrelizumab, Sintilimab, Cemiplimab, Envafolimab, BMS-936559, JS003, SHR-1316, GS-4224, AN-4005, or MX-10181.
28. The kit or composition according to any one of embodiments 18-26, wherein the immune checkpoint inhibitor is a PD-1/PD-L1 small molecule inhibitor, optionally, the PD-1/PD-L1 small molecule inhibitor is INCB-086550, Lazertinib, IMMH-010, CA-170, ABSK043, or RRx-001.
29. The kit or composition according to any one of embodiments 18-26, wherein the immune checkpoint inhibitor is a TIGIT inhibitor, optionally, the TIGIT inhibitor is Ociperlimab (BGB-A1217), Vibostolimab, Domvanalimab (AB154), Tiragolumab, Belrestotug, Etigilimab, ONO-4686, JS-006, AZD-2936, HLX-301, SEA-TGT, M-6223, IBI-939, COM-902, AB-308, AGEN-1777, AK-127, BAT-6021, BAT-6005, ASP-8374, PM-1022, BMS-986207, HB0036, or IBI-321.
30. The kit or composition according to embodiment 18, wherein the FAK inhibitor is IN10018 or a pharmaceutically acceptable salt thereof, the microtubule inhibitor is Docetaxel; and the immune checkpoint inhibitor is an anti-PD-1/PD-L1 antibody, or a PD-1/PD-L1 small molecule inhibitor, alternatively, the FAK inhibitor is IN10018 or a pharmaceutically acceptable salt thereof, the microtubule inhibitor is Docetaxel; the immune checkpoint inhibitor is an anti-PD-1/PD-L1 antibody.
31. The kit or composition according to embodiment 18, wherein the FAK inhibitor is IN10018 or a pharmaceutically acceptable salt thereof, the microtubule inhibitor is Paclitaxel, and the immune checkpoint inhibitor is an anti-PD-1/PD-L1 antibody, or a PD-1/PD-L1 small molecule inhibitor, alternatively, the FAK inhibitor is IN10018 or a pharmaceutically acceptable salt thereof, the microtubule inhibitor is Paclitaxel; the immune checkpoint inhibitor is an anti-PD-1/PD-L1 antibody.
32. The kit or composition according to any one of embodiments 18-31, wherein the composition is used as a medicament.
33. The kit or composition according to embodiment 32, wherein the medicament is used for treating a tumor, wherein the tumor is bladder cancer, breast cancer, cervical cancer, colon cancer (including colorectal cancer), esophageal cancer, esophageal squamous cell carcinoma, head and neck cancer, liver cancer, lung cancer (including small cell lung cancer and non-small cell lung cancer), melanoma, myeloma, rhabdomyosarcoma, inflammatory myofibroblastoma, neuroblastoma, pancreatic cancer, prostate cancer, kidney cancer, renal cell carcinoma, sarcoma (including osteosarcoma), skin cancer (including squamous cell carcinoma), gastric cancer, testicular cancer, thyroid cancer, uterine cancer, mesothelioma, cholangiocarcinoma, leiomyosarcoma, liposarcoma, nasopharyngeal carcinoma, neuroendocrine carcinoma, ovarian carcinoma, salivary adenocarcinoma, metastases caused by spindle cell carcinoma, anaplastic large cell lymphoma, undifferentiated thyroid cancer, non-Hodgkin's lymphoma, Hodgkin's lymphoma, glioma or hematologic malignancies such as acute myeloid leukemia (AML), acute lymphocytic leukemia (ALL), diffuse large B-cell lymphoma (DLBCL), follicular lymphoma (FL), chronic lymphocytic leukemia (CLL), or chronic myelogenous leukemia (CML); alternatively, the tumor is breast cancer, ovarian cancer, colon cancer (including colorectal cancer), lung cancer (including small cell lung cancer and non-small cell lung cancer), melanoma, or pancreatic cancer.
34. The kit or composition according to embodiment 33, wherein the tumor is breast cancer, ovarian cancer, or colon cancer (including colorectal cancer).
35. A method for treating a tumor in a subject, wherein the method comprises administering to the subject a therapeutically effective amount of an FAK inhibitor, a microtubule inhibitor and an immune checkpoint inhibitor.
36. The method according to embodiment 35, wherein the FAK inhibitor is IN10018, Defactinib, GSK2256098, PF-00562271, VS-4718, APG-2449, AMP945, AMP886, or a pharmaceutically acceptable salt thereof, alternatively, the FAK inhibitor is IN10018, Defactinib, AMP945 or a pharmaceutically acceptable salt thereof, yet alternatively, the FAK inhibitor is IN10018, or a pharmaceutically acceptable salt thereof, alternatively, the FAK inhibitor is IN10018 tartrate, wherein the IN10018 has a structure of: 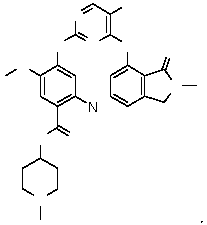
37. The method according to any one of embodiments 35-36, wherein the microtubule inhibitor is a taxane, Eribulin, Ixempra or a vinca alkaloid.
38. The method according to any one of embodiments 35-37, wherein the microtubule inhibitor is a taxane.
39. The method according to any one of embodiments 37-38, wherein the taxane is Docetaxel, Paclitaxel, Cabazitaxel or Cephalomannine, alternatively Docetaxel or Paclitaxel.
40. The method according to any one of embodiments 35-39, wherein the microtubule inhibitor is Docetaxel.
41. The method according to any one of embodiments 35-39, wherein the microtubule inhibitor is Paclitaxel.
42. The method according to any one of embodiments 35-37, wherein the microtubule inhibitor is Eribulin.
43. The method according to any one of embodiments 35-42, wherein the immune checkpoint inhibitor is an anti-PD-1/PD-L1 antibody, a PD-1/PD-L1 small molecule inhibitor or a TIGIT inhibitor.
44. The method according to any one of embodiments 35-43, wherein the immune checkpoint inhibitor is an anti-PD-1/PD-L1 antibody, optionally, the anti-PD-1/PD-L1 antibody is Pembrolizumab, Tislelizumab, Nivolumab, Toripalimab, Atezolizumab, Durvalumab, Avelumab, Camrelizumab, Sintilimab, Cemiplimab, Envafolimab, BMS-936559, JS003, SHR-1316, GS-4224, AN-4005, or MX-10181.
45. The method according to any one of embodiments 35-43, wherein the immune checkpoint inhibitor is a PD-1/PD-L1 small molecule inhibitor, optionally, the PD-1/PD-L1 small molecule inhibitor is INCB-086550, Lazertinib, IMMH-010, CA-170, ABSK043, or RRx-001.
46. The method according to any one of embodiments 35-43, wherein the immune checkpoint inhibitor is a TIGIT inhibitor, optionally, the TIGIT inhibitor is Ociperlimab (BGB-A1217), Vibostolimab, Domvanalimab (AB154), Tiragolumab, Belrestotug, Etigilimab, ONO-4686, JS-006, AZD-2936, HLX-301, SEA-TGT, M-6223, IBI-939, COM-902, AB-308, AGEN-1777, AK-127, BAT-6021, BAT-6005, ASP-8374, PM-1022, BMS-986207, HB0036, or IBI-321.
47. The method according to embodiment 35, wherein the FAK inhibitor is IN10018 or a pharmaceutically acceptable salt thereof, the microtubule inhibitor is Docetaxel; and the immune checkpoint inhibitor is an anti-PD-1/PD-L1 antibody, or a PD-1/PD-L1 small molecule inhibitor, alternatively, the FAK inhibitor is IN10018 or a pharmaceutically acceptable salt thereof, the microtubule inhibitor is Docetaxel; the immune checkpoint inhibitor is an anti-PD-1/PD-L1 antibody.
48. The method according to embodiment 35, wherein the FAK inhibitor is IN10018 or a pharmaceutically acceptable salt thereof, the microtubule inhibitor is Paclitaxel, and the immune checkpoint inhibitor is an anti-PD-1/PD-L1 antibody, or a PD-1/PD-L1 small molecule inhibitor, alternatively, the FAK inhibitor is IN10018 or a pharmaceutically acceptable salt thereof, the microtubule inhibitor is Paclitaxel; the immune checkpoint inhibitor is an anti-PD-1/PD-L1 antibody.
49. The method according to any one of embodiments 35-48, wherein the FAK inhibitor, the microtubule inhibitor and the immune checkpoint inhibitor are administered simultaneously or sequentially to the subject.
50. The method according to any one of embodiments 35-49, wherein the tumor is bladder cancer, breast cancer, cervical cancer, colon cancer (including colorectal cancer), esophageal cancer, esophageal squamous cell carcinoma, head and neck cancer, liver cancer, lung cancer (including small cell lung cancer and non-small cell lung cancer), melanoma, myeloma, rhabdomyosarcoma, inflammatory myofibroblastoma, neuroblastoma, pancreatic cancer, prostate cancer, kidney cancer, renal cell carcinoma, sarcoma (including osteosarcoma), skin cancer (including squamous cell carcinoma), gastric cancer, testicular cancer, thyroid cancer, uterine cancer, mesothelioma, cholangiocarcinoma, leiomyosarcoma, liposarcoma, nasopharyngeal carcinoma, neuroendocrine carcinoma, ovarian carcinoma, salivary adenocarcinoma, metastases caused by spindle cell carcinoma, anaplastic large cell lymphoma, undifferentiated thyroid cancer, non-Hodgkin's lymphoma, Hodgkin's lymphoma, glioma or hematologic malignancies such as acute myeloid leukemia (AML), acute lymphocytic leukemia (ALL), diffuse large B-cell lymphoma (DLBCL), follicular lymphoma (FL), chronic lymphocytic leukemia (CLL), or chronic myelogenous leukemia (CML); alternatively, the tumor is breast cancer, ovarian cancer, colon cancer (including colorectal cancer), lung cancer (including small cell lung cancer and non-small cell lung cancer), melanoma, or pancreatic cancer.
51. The method according to any one of embodiments 35-50, wherein the tumor is breast cancer, ovarian cancer, or colon cancer (including colorectal cancer).
52. An FAK inhibitor, a microtubule inhibitor and an immune checkpoint inhibitor, for use in a method of treating a tumor by increasing immunogenic cell death in a subject.
53. The FAK inhibitor, the microtubule inhibitor and the immune checkpoint inhibitor according to embodiment 52, wherein the FAK inhibitor is IN10018, Defactinib, GSK2256098, PF-00562271, VS-4718, APG-2449, AMP945, AMP886, or a pharmaceutically acceptable salt thereof, alternatively, the FAK inhibitor is IN10018, Defactinib, AMP945, or a pharmaceutically acceptable salt thereof, and yet alternatively, the FAK inhibitor is IN10018, or a pharmaceutically acceptable salt thereof, alternatively, the FAK inhibitor is IN10018 tartrate, wherein the IN10018 has a structure of: 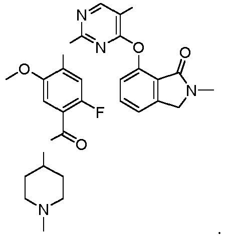
54. The FAK inhibitor, the microtubule inhibitor and the immune checkpoint inhibitor according to any one of embodiments 52-53, wherein the microtubule inhibitor is a taxane, Eribulin, Ixempra or a vinca alkaloid.
55. The FAK inhibitor, the microtubule inhibitor and the immune checkpoint inhibitor according to any one of embodiments 52-54, wherein the microtubule inhibitor is a taxane.
56. The FAK inhibitor, the microtubule inhibitor and the immune checkpoint inhibitor according to any one of embodiments 54-55, wherein the taxane is Docetaxel, Paclitaxel, Cabazitaxel or Cephalomannine, alternatively Docetaxel or Paclitaxel.
57. The FAK inhibitor, the microtubule inhibitor and the immune checkpoint inhibitor according to any one of embodiments 52-56, wherein the microtubule inhibitor is Docetaxel.
58. The FAK inhibitor, the microtubule inhibitor and the immune checkpoint inhibitor according to any one of embodiments 52-56, wherein the microtubule inhibitor is Paclitaxel.
59. The FAK inhibitor, the microtubule inhibitor and the immune checkpoint inhibitor according to any one of embodiments 52-54, wherein the microtubule inhibitor is Eribulin.
60. The FAK inhibitor, the microtubule inhibitor and the immune checkpoint inhibitor according to any one of embodiments 52-59, wherein the immune checkpoint inhibitor is an anti-PD-1/PD-L1 antibody, a PD-1/PD-L1 small molecule inhibitor, or a TIGIT inhibitor.
61. The FAK inhibitor, the microtubule inhibitor and the immune checkpoint inhibitor according to any one of embodiments 52-60, wherein the immune checkpoint inhibitor is an anti-PD-1/PD-L1 antibody, optionally, the anti-PD-1/PD-L1 antibody is Pembrolizumab, Tislelizumab, Nivolumab, Toripalimab, Atezolizumab, Durvalumab, Avelumab, Camrelizumab, Sintilimab, Cemiplimab, Envafolimab, BMS-936559, JS003, SHR-1316, GS-4224, AN-4005, or MX-10181.
62. The FAK inhibitor, the microtubule inhibitor and the immune checkpoint inhibitor according to any one of embodiments 52-60, wherein the immune checkpoint inhibitor is a PD-1/PD-L1 small molecule inhibitor, optionally, the PD-1/PD-L1 small molecule inhibitor is INCB-086550, Lazertinib, IMMH-010, CA-170, ABSK043, or RRx-001.
63. The FAK inhibitor, the microtubule inhibitor and the immune checkpoint inhibitor according to any one of embodiments 52-60, wherein the immune checkpoint inhibitor is a TIGIT inhibitor, optionally, the TIGIT inhibitor is Ociperlimab (BGB-A1217), Vibostolimab, Domvanalimab (AB154), Tiragolumab, Belrestotug, Etigilimab, ONO-4686, JS-006, AZD-2936, HLX-301, SEA-TGT, M-6223, IBI-939, COM-902, AB-308, AGEN-1777, AK-127, BAT-6021, BAT-6005, ASP-8374, PM-1022, BMS-986207, HB0036, or IBI-321.
64. The FAK inhibitor, the microtubule inhibitor and the immune checkpoint inhibitor according to embodiment 52, wherein the FAK inhibitor is IN10018 or a pharmaceutically acceptable salt thereof, the microtubule inhibitor is Docetaxel; and the immune checkpoint inhibitor is an anti-PD-1/PD-L1 antibody, or a PD-1/PD-L1 small molecule inhibitor, alternatively, the FAK inhibitor is IN10018 or a pharmaceutically acceptable salt thereof, the microtubule inhibitor is Docetaxel; the immune checkpoint inhibitor is an anti-PD-1/PD-L1 antibody.
65. The FAK inhibitor, the microtubule inhibitor and the immune checkpoint inhibitor according to embodiment 52, wherein the FAK inhibitor is IN10018 or a pharmaceutically acceptable salt thereof, the microtubule inhibitor is Paclitaxel, and the immune checkpoint inhibitor is an anti-PD-1/PD-L1 antibody, or a PD-1/PD-L1 small molecule inhibitor, alternatively, the FAK inhibitor is IN10018 or a pharmaceutically acceptable salt thereof, the microtubule inhibitor is Paclitaxel; the immune checkpoint inhibitor is an anti-PD-1/PD-L1 antibody.
66. The FAK inhibitor, the microtubule inhibitor and the immune checkpoint inhibitor according to any one of embodiments 52-65, wherein the FAK inhibitor, the microtubule inhibitor and the immune checkpoint inhibitor are administered simultaneously or sequentially to the subject.
67. The FAK inhibitor, the microtubule inhibitor and the immune checkpoint inhibitor according to any one of embodiments 52-66, wherein the tumor is bladder cancer, breast cancer, cervical cancer, colon cancer (including colorectal cancer), esophageal cancer, esophageal squamous cell carcinoma, head and neck cancer, liver cancer, lung cancer (including small cell lung cancer and non-small cell lung cancer), melanoma, myeloma, rhabdomyosarcoma, inflammatory myofibroblastoma, neuroblastoma, pancreatic cancer, prostate cancer, kidney cancer, renal cell carcinoma, sarcoma (including osteosarcoma), skin cancer (including squamous cell carcinoma), gastric cancer, testicular cancer, thyroid cancer, uterine cancer, mesothelioma, cholangiocarcinoma, leiomyosarcoma, liposarcoma, nasopharyngeal carcinoma, neuroendocrine carcinoma, ovarian carcinoma, salivary adenocarcinoma, metastases caused by spindle cell carcinoma, anaplastic large cell lymphoma, undifferentiated thyroid cancer, non-Hodgkin's lymphoma, Hodgkin's lymphoma, glioma or hematologic malignancies such as acute myeloid leukemia (AML), acute lymphocytic leukemia (ALL), diffuse large B-cell lymphoma (DLBCL), follicular lymphoma (FL), chronic lymphocytic leukemia (CLL), or chronic myelogenous leukemia (CML); alternatively, the tumor is breast cancer, ovarian cancer, colon cancer (including colorectal cancer), lung cancer (including small cell lung cancer and non-small cell lung cancer), melanoma, or pancreatic cancer.
68. The FAK inhibitor, the microtubule inhibitor and the immune checkpoint inhibitor according to any one of embodiments 52-67, wherein the tumor is breast cancer, ovarian cancer, or colon cancer (including colorectal cancer).
69. A method for treating a tumor by increasing immunogenic cell death in a subject, wherein the method comprises administering to the subject a therapeutically effective amount of an FAK inhibitor, a microtubule inhibitor and an immune checkpoint inhibitor.
70. The method according to embodiment 69, wherein the FAK inhibitor is IN10018, Defactinib, GSK2256098, PF-00562271, VS-4718, APG-2449, AMP945, AMP886, or a pharmaceutically acceptable salt thereof, alternatively, the FAK inhibitor is IN10018, Defactinib, AMP945, or a pharmaceutically acceptable salt thereof, and yet alternatively, the FAK inhibitor is IN10018, or a pharmaceutically acceptable salt thereof, alternatively, the FAK inhibitor is IN10018 tartrate, wherein the IN10018 has a structure of: 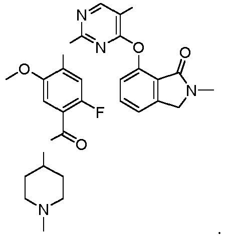
71. The method according to any one of embodiments 69-70, wherein the microtubule inhibitor is a taxane, Eribulin, Ixempra or a vinca alkaloid.
72. The method according to any one of embodiments 69-71, wherein the microtubule inhibitor is a taxane.
73. The method according to any one of embodiments 71-72, wherein the taxane is Docetaxel, Paclitaxel, Cabazitaxel or Cephalomannine, alternatively Docetaxel or Paclitaxel.
74. The method according to any one of embodiments 69-73, wherein the microtubule inhibitor is Docetaxel.
75. The method according to any one of embodiments 69-73, wherein the microtubule inhibitor is Paclitaxel.
76. The method according to any one of embodiments 69-73, wherein the microtubule inhibitor is Eribulin.
77. The method according to any one of embodiments 69-76, wherein the immune checkpoint inhibitor is an anti-PD-1/PD-L1 antibody, a PD-1/PD-L1 small molecule inhibitor, or a TIGIT inhibitor.
78. The method according to any one of embodiments 69-77, wherein the immune checkpoint inhibitor is an anti-PD-1/PD-L1 antibody, optionally, the anti-PD-1/PD-L1 antibody is Pembrolizumab, Tislelizumab, Nivolumab, Toripalimab, Atezolizumab, Durvalumab, Avelumab, Camrelizumab, Sintilimab, Cemiplimab, Envafolimab, BMS-936559, JS003, SHR-1316, GS-4224, AN-4005, or MX-10181.
79. The method according to any one of embodiments 69-77, wherein the immune checkpoint inhibitor is a PD-1/PD-L1 small molecule inhibitor, optionally, the PD-1/PD-L1 small molecule inhibitor is INCB-086550, Lazertinib, IMMH-010, CA-170, ABSK043, or RRx-001.
80. The method according to any one of embodiments 69-77, wherein the immune checkpoint inhibitor is a TIGIT inhibitor, optionally, the TIGIT inhibitor is Ociperlimab (BGB-A1217), Vibostolimab, Domvanalimab (AB154), Tiragolumab, Belrestotug, Etigilimab, ONO-4686, JS-006, AZD-2936, HLX-301, SEA-TGT, M-6223, IBI-939, COM-902, AB-308, AGEN-1777, AK-127, BAT-6021, BAT-6005, ASP-8374, PM-1022, BMS-986207, HB0036, or IBI-321.
81. The method according to embodiment 69, wherein the FAK inhibitor is IN10018 or a pharmaceutically acceptable salt thereof, the microtubule inhibitor is Docetaxel; and the immune checkpoint inhibitor is an anti-PD-1/PD-L1 antibody, or a PD-1/PD-L1 small molecule inhibitor, alternatively, the FAK inhibitor is IN10018 or a pharmaceutically acceptable salt thereof, the microtubule inhibitor is Docetaxel; the immune checkpoint inhibitor is an anti-PD-1/PD-L1 antibody.
82. The method according to embodiment 69, wherein the FAK inhibitor is IN10018 or a pharmaceutically acceptable salt thereof, the microtubule inhibitor is Paclitaxel, and the immune checkpoint inhibitor is an anti-PD-1/PD-L1 antibody, or a PD-1/PD-L1 small molecule inhibitor, alternatively, the FAK inhibitor is IN10018 or a pharmaceutically acceptable salt thereof, the microtubule inhibitor is Paclitaxel; the immune checkpoint inhibitor is an anti-PD-1/PD-L1 antibody.
83. The method according to any one of embodiments 69-82, wherein the FAK inhibitor, the microtubule inhibitor, and the immune checkpoint inhibitor are administered simultaneously or sequentially to the subject.
84. The method according to any one of embodiments 69-83, wherein the tumor is bladder cancer, breast cancer, cervical cancer, colon cancer (including colorectal cancer), esophageal cancer, esophageal squamous cell carcinoma, head and neck cancer, liver cancer, lung cancer (including small cell lung cancer and non-small cell lung cancer), melanoma, myeloma, rhabdomyosarcoma, inflammatory myofibroblastoma, neuroblastoma, pancreatic cancer, prostate cancer, kidney cancer, renal cell carcinoma, sarcoma (including osteosarcoma), skin cancer (including squamous cell carcinoma), gastric cancer, testicular cancer, thyroid cancer, uterine cancer, mesothelioma, cholangiocarcinoma, leiomyosarcoma, liposarcoma, nasopharyngeal carcinoma, neuroendocrine carcinoma, ovarian carcinoma, salivary adenocarcinoma, metastases caused by spindle cell carcinoma, anaplastic large cell lymphoma, undifferentiated thyroid cancer, non-Hodgkin's lymphoma, Hodgkin's lymphoma, glioma or hematologic malignancies such as acute myeloid leukemia (AML), acute lymphocytic leukemia (ALL), diffuse large B-cell lymphoma (DLBCL), follicular lymphoma (FL), chronic lymphocytic leukemia (CLL), or chronic myelogenous leukemia (CML); alternatively, the tumor is breast cancer, ovarian cancer, colon cancer (including colorectal cancer), lung cancer (including small cell lung cancer and non-small cell lung cancer), melanoma, or pancreatic cancer.
85. The method according to any one of embodiments 69-84, wherein the tumor is breast cancer, ovarian cancer, or colon cancer (including colorectal cancer).
86. Use of an FAK inhibitor in the manufacture of a medicament for treating a tumor in a subject, wherein the FAK inhibitor, a microtubule inhibitor and an immune checkpoint inhibitor are administered to the subject.
87. Use of a microtubule inhibitor in the manufacture of a medicament for treating a tumor in a subject, wherein an FAK inhibitor, the microtubule inhibitor and an immune checkpoint inhibitor are administered to the subject.
88. Use of an immune checkpoint inhibitor in the manufacture of a medicament for treating a tumor in a subject, wherein an FAK inhibitor, a microtubule inhibitor, and the immune checkpoint inhibitor are administered to the subject.
89. Use of an FAK inhibitor, a microtubule inhibitor and an immune checkpoint inhibitor in the manufacture of a combined medicament for treating a tumor.
90. Use of an FAK inhibitor in the manufacture of a combined medicament with a microtubule inhibitor and an immune checkpoint inhibitor for treating a tumor.
91. Use of a microtubule inhibitor in the manufacture of a combined medicament with an FAK inhibitor and an immune checkpoint inhibitor for treating a tumor.
92. Use of an immune checkpoint inhibitor in the manufacture of a combined medicament with an FAK inhibitor and a microtubule inhibitor for treating a tumor.
93. Use of an FAK inhibitor, a microtubule inhibitor and an immune checkpoint inhibitor in the manufacture of a medicament for combined treatment of a tumor.
94. Use of an FAK inhibitor in the manufacture of a medicament for combined treatment of a tumor with a microtubule inhibitor and an immune checkpoint inhibitor.
95. Use of a microtubule inhibitor in the manufacture of a medicament for combined treatment of a tumor with an FAK inhibitor and an immune checkpoint inhibitor.
96. Use of an immune checkpoint inhibitor in the manufacture of a medicament for combined treatment of a tumor with an FAK inhibitor and a microtubule inhibitor.
97. The use according to any one of embodiments 86-96, wherein the FAK inhibitor is IN10018, Defactinib, GSK2256098, PF-00562271, VS-4718, APG-2449, AMP945, AMP886 or a pharmaceutically acceptable salt thereof, alternatively IN10018, Defactinib, AMP945 or a pharmaceutically acceptable salt thereof, yet alternatively IN10018 or a pharmaceutically acceptable salt thereof, alternatively IN10018 tartrate, wherein the structure of IN10018 is: 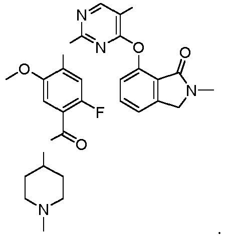
98. The use according to any one of embodiments 86-97, wherein the microtubule inhibitor is a taxane, Eribulin, Ixempra or a vinca alkaloid.
99. The use according to any one of embodiments 86-98, wherein the microtubule inhibitor is a taxane.
100. The use according to any one of embodiments 98-99, wherein the taxane is Docetaxel, Paclitaxel, Cabazitaxel or Cephalomannine, alternatively Docetaxel or Paclitaxel.
101. The use according to any one of embodiments 98-100, wherein the microtubule inhibitor is Docetaxel.
102. The use according to any one of embodiments 98-100, wherein the microtubule inhibitor is Paclitaxel.
103. The use according to any one of embodiments 98-100, wherein the microtubule inhibitor is Eribulin.
104. The use according to any one of embodiments 98-103, wherein the immune checkpoint inhibitor is an anti-PD-1/PD-L1 antibody, a PD-1/PD-L1 small molecule inhibitor or a TIGIT inhibitor.
105. The use according to any one of embodiments 98-104, wherein the immune checkpoint inhibitor is an anti-PD-1/PD-L1 antibody, and optionally the anti-PD-1/PD-L1 antibody is Pembrolizumab, Tislelizumab, Nivolumab, Toripalimab, Atezolizumab, Durvalumab, Avelumab, Camrelizumab, Sintilimab, Cemiplimab, Envafolimab, BMS-936559, JS003, SHR-1316, GS-4224, AN-4005, or MX-10181.
106. The use according to any one of embodiments 98-104, wherein the immune checkpoint inhibitor is a PD-1/PD-L1 small molecule inhibitor, and optionally the PD-1/PD-L1 small molecule inhibitor is INCB-086550, Lazertinib, IMMH-010, CA-170, ABSK043 or RRx-001.
107. The use according to any one of embodiments 98-104, wherein the immune checkpoint inhibitor is a TIGIT inhibitor, and optionally, the TIGIT inhibitor is Ociperlimab (BGB-A1217), Vibostolimab, Domvanalimab (AB154), Tiragolumab, Belrestotug, Etigilimab, ONO-4686, JS-006, AZD-2936, HLX-301, SEA-TGT, M-6223, IBI-939, COM-902, AB-308, AGEN-1777, AK-127, BAT-6021, BAT-6005, ASP-8374, PM-1022, BMS-986207, HB0036, or IBI-321.
108. The use according to any one of embodiments 98-107, wherein the FAK inhibitor is IN10018 or a pharmaceutically acceptable salt thereof, the microtubule inhibitor is Docetaxel, and the immune checkpoint inhibitor is an anti-PD-1/PD-L1 antibody or a PD-1/PD-L1 small molecule inhibitor, and alternatively, the FAK inhibitor is IN10018 or a pharmaceutically acceptable salt thereof, the microtubule inhibitor is Docetaxel; the immune checkpoint inhibitor is an anti-PD-1/PD-L1 antibody.
109. The use according to any one of embodiments 98-107, wherein the FAK inhibitor is IN10018 or a pharmaceutically acceptable salt thereof, the microtubule inhibitor is Paclitaxel, and the immune checkpoint inhibitor is an anti-PD-1/PD-L1 antibody or a PD-1/PD-L1 small molecule inhibitor, and alternatively, the FAK inhibitor is IN10018 or a pharmaceutically acceptable salt thereof, the microtubule inhibitor is Paclitaxel; the immune checkpoint inhibitor is an anti-PD-1/PD-L1 antibody.
110. The use according to any one of embodiments 98-109, wherein the FAK inhibitor, the microtubule inhibitor and the immune checkpoint inhibitor are administered simultaneously or sequentially to the subject.
111. The use according to any one of embodiments 86-110, wherein the tumor is bladder cancer, breast cancer, cervical cancer, colon cancer (including colorectal cancer), esophageal cancer, esophageal squamous cell carcinoma, head and neck cancer, liver cancer, lung cancer (including small cell lung cancer and non-small cell lung cancer), melanoma, myeloma, rhabdomyosarcoma, inflammatory myofibroblastoma, neuroblastoma, pancreatic cancer, prostate cancer, kidney cancer, renal cell carcinoma, sarcoma (including osteosarcoma), skin cancer (including squamous cell carcinoma), gastric cancer, testicular cancer, thyroid cancer, uterine cancer, mesothelioma, cholangiocarcinoma, leiomyosarcoma, liposarcoma, nasopharyngeal carcinoma, neuroendocrine carcinoma, ovarian carcinoma, salivary adenocarcinoma, metastases caused by spindle cell carcinoma, anaplastic large cell lymphoma, undifferentiated thyroid cancer, non-Hodgkin's lymphoma, Hodgkin's lymphoma, glioma or hematologic malignancies such as acute myeloid leukemia (AML), acute lymphocytic leukemia (ALL), diffuse large B-cell lymphoma (DLBCL), follicular lymphoma (FL), chronic lymphocytic leukemia (CLL), or chronic myelogenous leukemia (CML); alternatively, the tumor is breast cancer, ovarian cancer, colon cancer (including colorectal cancer), lung cancer (including small cell lung cancer and non-small cell lung cancer), melanoma, or pancreatic cancer.
112. The use according to any one of embodiments 98-111, wherein the tumor is breast cancer, ovarian cancer, or colon cancer (including colorectal cancer).

### Examples

The following examples are provided to further illustrate the present disclosure. It should be understood that these examples are only used to illustrate the present disclosure and are not used to limit the scope of the present disclosure.

The assay methods without specific conditions in the following examples can be carried out according to the conventional conditions of this kind of reaction or according to the conditions suggested by the manufacturer. The assay materials and reagents used in the following examples are all commercially available unless otherwise specified.

The abbreviations used in the examples have the following meanings:

| Abbreviation | Name |
|---|---|
| siRNA | Small interfering RNA |
| DMSO | Dimethyl sulfoxide |
| COMBO | Combination |
| HMGB1 | High mobility group box-1 protein |
| AAALAC | Association for Assessment and Accreditation of Laboratory Animal Care |
| IACUC | Institutional Animal Care and Use Committee |
| SPF | Specific pathogen Free |
| BIW | Twice a week |
| BID | Twice a day |
| BW | Body weight |
| EDTA | Ethylenediamine tetraacetic acid |
| GLP | Good Laboratory Practice for Non-clinical Laboratory Studies |
| *p.o*. | Intragastric administration |
| *i*.*v*. | Intravenous injection administration |
| *i.p.* | Intraperitoneal injection administration |
| DPBS | Dulbecco's Phosphate Buffered Saline |
| DDH2O | Double distilled water |
| RT | Room temperature |
| SEM | Standard error of the mean |
| TGI | Tumor growth inhibition rate |
| TV | Tumor volume |
| TW | Tumor weight |
| FBS | Fetal bovine serum |
| PBS | Phosphate-buffered saline |
| MC | Methylcellulose |
| CO₂ | Carbon dioxide |
| CRT | Calreticulin |
| Annexin-V-FITC | Fluorescein isothiocyanate-labeled annexin |
| PI | Propidium iodide |

### Example 1: Study on the enhancement of immunogenic cell death of ovarian cancer cells in response to Docetaxel by FAK target inhibition

### Assay protocol:

1. FAK silencing combined with Docetaxel promotes apoptosis of ovarian cancer cells HCC827
   The siRNA technology was used in the in vitro assay to reduce the expression level of FAK, and combined with Docetaxel for 48 hours; flow cytometry was used to detect apoptosis in control (DMSO) and FAK silenced cells
2. FAK silencing combined with Docetaxel can effectively promote the production of ICD in ovarian cancer cells SK-OV-3
   The siRNA technology was used in the in vitro assay to reduce the expression level of FAK, and combined with Docetaxel for 48 hours. Flow cytometry was used to detect the expression of Calreticulin, the main target of ICD.

Assay **antibodies:** recombinant Alexa Fluor^{®} 647 fluorescent Anti-Calreticulin antibody (Abcam, ab196159), Annexin V apoptosis detection kit (Invitrogen, A35110).

FAK siRNA was provided by Gene Pharma, and the sequence is shown in the following table:
F: CCUGUAUGCCUAUCAGCUUTT;
R: AAGCUGAUAGGCAUACAGGTT

### Assay instruments:

Fluorescence microscope (Olympus U-HGLGPS). Chemiluminescence imager (BIORAD chemidoc touch)

### Assay results:

1. Combining FAK silencing with Docetaxel significantly increased the apoptosis of SK-OV-3 cells
   SK-OV-3 cells were transfected with control siRNA or FAK siRNA at a final concentration of 50 nM, respectively. After the transfection for 24 hours, the cells were treated with 0.3 µM or 1 µM of Docetaxel for 48 hours, respectively. The cells were stained using Annexin V kit and detected by flow cytometry. The apoptotic values were counted, compared with those of the control group, and plotted using Graphpad 8.0. The results showed that compared with the control group, the apoptosis of the FAK silencing group was significantly enhanced after Docetaxel treatment, as shown in Fig. 1.
2. Combining FAK silencing with Docetaxel significantly increased the release and exposure of Calreticulin, the ICD target
   SK-OV-3 cells were transfected with control siRNA or FAK siRNA at a final concentration of 50 nM, respectively. After the transfection for 24 hours, the cells were treated with 1 µM of Docetaxel for 48 hours. The cells were fluorescent stained with Calreticulin antibody, and the staining results were analyzed by flow cytometry. FlowJo software result statistics showed that after FAK silencing combined with Docetaxel, the release and exposure of Calreticulin were significantly enhanced, as shown in Fig. 2.

### Example 2: Study on the enhancement of immunogenic cell death of breast cancer cells in response to Eribulin by FAK target inhibition

### Assay protocol:

1. FAK silencing combined with Eribulin promotes apoptosis of breast cancer cells MDA-MB-231
   The siRNA technology was used in the in vitro assays to reduce the expression level of FAK, and combined with Eribulin for 48 hours; flow cytometry was used to detect apoptosis in control and FAK silenced cells.
2. FAK silencing combined with Eribulin can effectively promote the production of ICD in breast cancer cells MDA-MB-231
   The siRNA technology was used in the in vitro assay to reduce the expression level of FAK, and combined with Eribulin for 48 hours. Flow cytometry was used to detect the expression of Calreticulin, the main target of ICD.

### Assay antibodies:

Recombinant Alexa Fluor^{®} 647 fluorescent Anti-Calreticulin antibody (Abcam, ab196159), Annexin V apoptosis detection kit (Invitrogen, A35110).

FAK siRNA was provided by Gene Pharma, and the sequence is shown in the following table:
F: CCUGUAUGCCUAUCAGCUUTT;
R: AAGCUGAUAGGCAUACAGGTT
Assay instruments:
   Fluorescence microscope (Olympus U-HGLGPS). Chemiluminescence imager (BIORAD chemidoc touch).

### Assay results:

1. Combining FAK silencing with Eribulin significantly increased the apoptosis of MDA-MB-231 cells
   MDA-MB-231 cells were transfected with control siRNA or FAK siRNA at a final concentration of 50 nM, respectively. After the transfection for 24 hours, the cells were treated with 0.15 µM of Eribulin for 48 hours. The cells were stained using Annexin V kit and detected by flow cytometry. The early and late apoptosis values were counted, compared with those of the DMSO control group, and plotted using Graphpad 8.0. The results showed that compared with the control group, the early and late apoptosis of the FAK silencing group was significantly enhanced after Eribulin treatment, as shown in Fig. 3.
2. Combining FAK silencing with Eribulin significantly increased the release and exposure of Calreticulin, the ICD target
   MDA-MB-231 cells were transfected with control siRNA or FAK siRNA at a final concentration of 50 nM, respectively. After the transfection for 24 hours, the cells were treated with 0.15 µM of Eribulin for 48 hours. The cells were fluorescent stained with Calreticulin antibody, and the staining results were analyzed by flow cytometry. FlowJo software result statistics showed that after FAK silencing combined with Eribulin, the release and exposure of Calreticulin were significantly enhanced, as shown in Fig. 4.

### Example 3: Synergistic effect of IN10018 and Docetaxel in colon cancer CT26 cells

**CT26** cells were cultured with RPMI-1640 (Shanghai Basalmedia, catalog number: L210KJ, batch number: F210916) + 10% FBS (Gibco, catalog number: 10099-141c, batch number: 2158737cp), and passaged twice. When the cells were in good condition, they were placed in a 96-well plate, 3000 cells/well. After the cells were spread for 24 hours, the culture medium containing Docetaxel was added. 10 drug concentrations were set. The first concentration was 30 µM, which was serially diluted 3-fold, and the last one was the control, with the drug concentration of 0. Three compound wells were set for each drug concentration. At the same time, another group of cells was established with the same drug concentration as that in the above method. The difference was that: 5 µM IN10018 was added to each well, the drugs were mixed, and the plate was cultured in a 5% CO₂ incubator at 37 °C for 72 hours.

After 72 hours of drug action, the cells were observed under a microscope. 10 µl of CCK8 detection reagent (Cellorlab, classification number: CX001M, batch number: 2571100) was added to each well, and the plate was incubated in a 5% CO₂ incubator at 37 °C for 2-4 hours. Then the plate was read using microplate reader OD450 (chemiluminescence method).

The analysis results showed that the IC50 of the Docetaxel group was 0.19 µM; the IC50 of the Docetaxel + 5 µM IN10018 group was <0.005 µM. The IC50 of the group containing IN10018 was significantly lower than that of the group without IN10018, indicating that the efficacy of the two-drug combination treatment group was better than that of the single-drug treatment group, as shown in Fig. 5.

### Example 4: Study on IN10018 and Docetaxel in colon cancer CT26 cells

CT26 cells (Institute of Cell Biology, Chinese Academy of Sciences) were cultured with RPMI-1640 (Shanghai Basalmedia, catalog number: L210KJ, batch number: F210916) + 10% FBS (Gibco, catalog number: 10099-141c, batch number: 2158737cp), and passaged twice. When the cells were in good condition, they were placed in a 24-well plate. After 24 hours of cell spreading, four groups were set up. The first group was the control group, in which culture medium was added; the second group was IN10018 at a concentration of 5µM; the third group was Docetaxel (MCE, classification number: HY-B0011, batch number: 111613) with a concentration of 0.1µM; and the fourth group was the combination group of IN10018 (5 µM) and Docetaxel (0.1µM). The drugs were mixed and cultured in a 5 % CO₂ incubator at 37°C for 48 hours.

After 48 hours of drug action, the cells were observed and photographed under a microscope. The photos were saved. Then the cells were collected for flow cytometry, and the cells were washed twice with flow buffer (PBS + 2% FBS). 0.5 µl of AF647 anti-calreticulin antibody (abcam, catalog number: ab196159, batch number: CR33676773) was added to each well, and fully mixed. The cells were incubated at 4 °C for 20 minutes in the dark. After 20 minutes, the cells were washed twice with flow buffer (PBS + 2% FBS). Cell apoptosis detection kit (Beyotime, catalog number: CL062L, batch number: 021921210811) was used. 195 µl of Annexin-V-FITC binding solution was added, and the cells were gently mixed. 5 µl of annexin-V-FITC antibody was added, and the mixture was gently mixed. 10 µl of PI dye was added and mixed. The mixture was incubated at room temperature in the dark for 15 minutes, and then analyzed on a flow cytometer.

The cells were observed under a microscope. The cell status of the Docetaxel single-drug group and the two-drug combination group were poor. The two-drug combination group was the worst, and had more cell death. The cell status of the control group and IN10018 group were good. The results showed that the CRT positive rate and Annexin V positive rate of the two-drug combination group were higher than those of the single-drug group, as shown in Fig. 6, Fig. 7a and Fig. 7b.

### Example 5: Study on the Induction of Immunogenic Cell Death Targets by Eribulin and IN10018 in Mouse Breast Cancer 4T1 Cells

The compound information is shown in Table 1.

**Table 1:**

| Drug Name | Storage conditions | source | Solvent |
|---|---|---|---|
| Eribulin | 4 °C | Beijing Chempion Biotechnology Co., Ltd | DMSO |
| IN10018 | 4 °C | Synthesized according to the method in patent WO2010058032 | DPBS |

The main reagent information of the assay is shown in Table 2.

**Table 2:**

| Reagent name | Manufacturer | Article Number | Batch number |
|---|---|---|---|
| DPBS | Shanghai Basalmedia | B210KJ | B210902 |
| **RPMI-1640** | Shanghai Basalmedia | L210KJ | F210916 |
| FBS | GIBCO | 10099-141c | 2158737cp |
| Trypsin | Shanghai Basalmedia | S310KJ | C210903 |
| Annexin V-Apoptosis Detection Kit | Beyotime | C1062L | 021921210811 |
| AF647 Anti-Calreticulin Antibody | Abcam | ab196159 | CR33676773 |
| CoraLite^{®}488-Conjugated GRP94 Polyclonal Antibody | Proteintech | CL488-14700 | 21006579 |

The assay design is shown in Table 3.

**Table 3:**

| No. | Drug | Cell | Staining method |
|---|---|---|---|
| 1 | Control group | 4T1 | 1. CRT/Annexin-V/PI |
| 2 | IN10018 (5µM) | | |
| 3 | Eribulin (0.15 µM) | | |
| 4 | Eribulin(0.15µM)+IN10018 (5µM) | | 2. GRP94 |
| 5 | Eribulin (0.45 µM) | | |
| 6 | Eribulin(0.45µM)+IN10018 (5 µM) | | |

### Cell culture:

4T1 cells were cultured in monolayer in vitro under the following conditions: RPMI-1640 medium with 10% fetal bovine serum, 37 °C, 5% CO₂. Trypsin was used for routine digestion and passage treatment two to three times a week. When the cells were in the exponential growth phase and the adherent confluence reached 80%-90%, the cells were harvested and plated.

4T1 cells were digested with trypsin and then collected and counted. Based on the counting results, the cells were diluted with RPMI-1640+10% FBS to a concentration of 50,000 cells per milliliter. Then the cells were plated in 12-well cell culture plates, with 2 ml of cell suspension per well, i.e., 100,000 cells. After plating, the cells were cultured in a 37°C and 5% CO₂ incubator.

### Adding the compound to be tested:

After 24 hours of plating, test compounds IN10018 and Eribulin were added to different wells, respectively. The grouping and drug concentrations are shown in Table 3.

### Collecting cells for flow cytometry:

After 48 hours of drug action, the cells were photographed using a microscope, and then trypsinized and collected for flow cytometry staining.

The cells were washed twice with flow buffer (DPBS + 2% FBS), and each group of cells was divided into two equal parts. In one part, 0.5 µL of AF647 Anti-Calreticulin Antibody was added to each well, and mixed. The cells were incubated at 4°C in the dark for 20 min. After that, 195 µL of Annexin-V-FITC binding solution was added after washing once with flow buffer. 5 µL of Annexin-V-FITC antibody was added after gently mixing the cells. After mixing well, 1.0 µL of PI dye was added and mixed. The cells were incubated at room temperature in the dark for 15 min, and then subjected to flow cytometry. In another part, 0.5 µL of CoraLite^{®}488-Conjugated GRP94 Polyclonal Antibody was added to each well, and mixed well. The cells were incubated at 4°C in the dark for 20 min, then washed twice with flow buffer, and resuspended with 200 µL of flow buffer. The cells were then subjected to flow cytometry.

### Data Analysis:

After the assay, the cell positive rate was analyzed by Flowjo (V10) software.

### Assay results:

After 48 hours of drug action, the cell status of each group was observed under a microscope. The cell death was obvious in the Eribulin single drug group, and the most significant cell death was in the combined drug group. The cell viability in the control group and IN10018 group was better. Flow cytometry analysis results showed that the positive rates of CRT, Annexin-V and GRP94 in the combined drug group were significantly higher than those in the single drug group. The relevant detection results are shown in Figs. 8a, 8b and 8c.

### Example 6: Study on the induction of immunogenic cell death targets by Eribulin and IN10018 in mouse ovarian epithelial carcinoma ID8 cells and human ovarian epithelial carcinoma TOV-21G cells in vitro

The information of compounds and main reagents is the same as that in Example 5.

The assay design is shown in Table 4.

**Table 4:**

| No. | Drug | Cell | Staining method |
|---|---|---|---|
| 1 | Control group | ID8/TOV-21G | CRT/Annexin-V/PI |
| 2 | IN10018 (10µM) | | |
| 3 | Eribulin (0.045 µM) | | |
| 4 | Eribulin(0.045µM)+IN10018 (10µM) | | |

### Cell culture:

ID8 cells and TOV-21G cells were cultured in monolayer in vitro. ID8 cells were cultured in DMEM medium with 10% fetal bovine serum. TOV-21G cells were cultured in RPMI-1640 medium with 10% fetal bovine serum. Both were cultured in a 37 °C, 5% CO₂ incubator. Trypsin was used for routine digestion and passage treatment two to three times a week. When the cells were in the exponential growth phase and the adherent confluence reached 80%-90%, the cells were harvested and plated.

ID8 cells and TOV-21G cells were digested with trypsin and then collected and counted. Based on the counting results, the cells were diluted with the corresponding complete culture medium to a concentration of 50,000 cells per milliliter. Then the cells were plated in 12-well cell culture plates, with 2 ml of cell suspension per well, i.e., 100,000 cells.

After plating, the cells were cultured in a 37°C and 5% CO₂ incubator.

### Adding the compound to be tested:

After 24 hours of plating, test compounds IN10018 and Eribulin were added to different wells, respectively. The grouping and drug concentrations are shown in Table 4.

### Collecting cells for flow cytometry:

After 48 hours of drug action, the cells were photographed using a microscope, and then trypsinized and collected for flow cytometry staining.

The cells were washed twice with flow buffer (DPBS + 2% FBS). 0.5 µL of AF647 Anti-Calreticulin Antibody was added to each well, and mixed. The cells were incubated at 4°C in the dark for 20 min. After washing once with flow buffer, 195 µL of Annexin-V-FITC binding solution was added, and the cells were gently mixed well. 5 µL of Annexin-V-FITC antibody was added. After further mixing, 10 µL of PI dye was added and mixed well. The cells were incubated at room temperature in the dark for 15 min. The cells were then subjected to flow cytometry.

### Data Analysis:

After the assay, the cell positive rate was analyzed by Flowjo (V10) software.

### Assay results:

The cell status was observed under a microscope. Both cell lines showed that cell death was obvious in the Eribulin single-drug group, and the most significant cell death was in the combined drug group. The cell viability in the control group and IN10018 group was better. Flow cytometry analysis results showed that the positive rates of CRT and Annexin V in the combined drug group were significantly higher than those in the single drug group. The assay results of the ID8 cell line are shown in Figs. 9a and 9b; the assay results of the TOV-21G cell line are shown in Figs. 10a and 10b.

### Example 7: Study on the Induction of immunogenic cell death targets by Docetaxel/Eribulin/Paclitaxel and Defactinib in mouse colon cancer CT26 cells in vitro

The compound information is shown in Table 5.

**Table 5:**

| Drug Name | Storage conditions | Source | Solvent |
|---|---|---|---|
| Docetaxel | 4 °C | MCE | DMSO |
| Eribulin | 4 °C | Beijing Chempion Pharmaceutical Technology Co., Ltd. | DMSO |
| Paclitaxel | 4 °C | MCE | DMSO |
| Defactinib | 4 °C | Shanghai Chaolan Chemical Technology Center | DMSO |

The main reagent information of the assay is shown in Table 2:

The assay design is shown in Table 6:

**Table 6:**

| No. | Drug | Cell | Staining method |
|---|---|---|---|
| 1 | Control group | CT26 | CRT/Annexin-V/PI |
| 2 | Defactinib (5 µM) | | |
| 3 | Docetaxel (0.3µM) | | |
| 4 | Docetaxel(0.3 µM)+Defactinib(5µM) | | |
| 5 | Eribulin (0.3 µM) | | |
| 6 | Eribulin(0.3µM)+Defactinib(5µM) | | |
| 7 | Paclitaxel (6 µM) | | |
| 8 | Paclitaxel(6µM)+Defactinib(5µM) | | |

### Cell culture:

CT26 cells were cultured in monolayer in vitro under the following conditions: RPMI-1640 medium with 10% fetal bovine serum, 37°C, 5% CO₂. Trypsin was used for routine digestion and passage treatment two to three times a week. When the cells were in the exponential growth phase and the adherent confluence reached 80%-90%, the cells were harvested and plated.

CT26 cells were digested with trypsin, and then the cells were collected and counted. Based on the counting results, the cells were diluted with the corresponding complete culture medium to a concentration of 50,000 cells per milliliter. Then the cells were plated in 12-well cell culture plates, with 2 ml of cell suspension per well, i.e., 100,000 cells.

After plating, the cells were cultured in a 37°C and 5% CO₂ incubator.

After 24 hours of plating, the test compounds Docetaxel, Eribulin, Paclitaxel and Defactinib were added to different wells, respectively. The grouping and drug concentrations are shown in Table 6.

After 48 hours of drug action, the cells were photographed using a microscope, and then trypsinized and collected for flow cytometry staining.

The cells were washed twice with flow buffer (DPBS + 2% FBS). 0.5 µL of AF647 Anti-Calreticulin Antibody was added to each well, and mixed well. The cells were incubated at 4°C in the dark for 20 min. After washing once with flow buffer, 195 µL of Annexin-V-FITC binding solution was added, and the cells were gently mixed well. 5 µL of Annexin-V-FITC antibody was added. After further mixing, 10 µL of PI dye was added and mixed well. The cells were incubated at room temperature in the dark for 15 min. The cells were then subjected to flow cytometry.

### Data Analysis:

After the assay, the cell positive rate was analyzed by Flowjo (V10) software.

### Assay results:

In this assay, the effects of Docetaxel/Eribulin/Paclitaxel alone and in combination with Defactinib on inducing the expression of immune cell death targets in CT26 cells under in vitro conditions was evaluated.

After 48 hours of drug action in each group, the cell status was observed under a microscope. The cell death in the Docetaxel/Eribulin/Paclitaxel single drug group was obvious, and the cell death in the combined drug group was the most significant. The cell viability in the control group was better, and the cell death in the Defactinib single drug group was more significant. Flow cytometry analysis results showed that the positive rates of CRT and Annexin V in the combined drug group were significantly higher than those in the single drug group. The assay results of Docetaxel are shown in Figs. 11a and 11b; the assay results of Eribulin are shown in Figs. 12a and 12b; the assay results of Paclitaxel are shown in Figs. 13a and 13b.

### Example 8: Study on the induction of immunogenic cell death targets by Docetaxel/Eribulin and Defactinib in mouse breast cancer 4T1 cells in vitro

The compound information is shown in Table 5 (without Paclitaxel), and the main reagent information is shown in Table 2.

The assay design is shown in Table 7:

**Table 7:**

| **No.** | **Drug** | **Cell** | **Staining method** |
|---|---|---|---|
| 1 | Control group | 4T1 | CRT/Annexin-V/PI |
| 2 | Defactinib (5 µM) | | |
| 3 | Docetaxel (0.3 µM) | | |
| 4 | Docetaxel(0.3 µM +Defactinib(5µM) | | |
| 5 | Eribulin (0.1 µM) | | |
| 6 | Eribulin(0.1 µM)+Defactinib(5 µM) | | |

### Cell culture:

4T1 cells were cultured in a monolayer in vitro in RPMI-1640 medium with 10% fetal bovine serum in a 37 °C, 5% CO₂ incubator. Trypsin was used for routine digestion and passage treatment two to three times a week. When the cells were in the exponential growth phase and the adherent confluence reached 80%-90%, the cells were harvested and plated.

4T1 cells were digested with trypsin, and then the cells were collected and counted. Based on the counting results, the cells were diluted with the corresponding complete culture medium to a concentration of 50,000 cells per milliliter. Then the cells were plated in 12-well cell culture plates, with 2 ml of cell suspension per well, i.e., 100,000 cells.

After plating, the cells were cultured in a 37°C and 5% CO₂ incubator.

After 24 hours of plating, the test compounds Defactinib, Docetaxel and Eribulin were added to different wells, respectively. The grouping and drug concentrations are shown in Table 7.

### Collecting cells for flow cytometry:

After 48 hours of drug action, the cells were photographed using a microscope, and then trypsinized and collected for flow cytometry staining.

The cells were washed twice with flow buffer (DPBS + 2% FBS). 0.5 µL of AF647 Anti-Calreticulin Antibody was added to each well, and mixed well. The cells were incubated at 4°C in the dark for 20 min. After washing once with flow buffer, 195 µL of Annexin-V-FITC binding solution was added, and the cells were gently mixed well. 5 µL of Annexin-V-FITC antibody was added. After further mixing, 10 µL of PI dye was added and mixed. The cells were incubated at room temperature in the dark for 15 min. The cells were then subjected to flow cytometry.

### Data Analysis:

After the assay, the cell positive rate was analyzed by Flowjo (V10) software.

### Assay results:

After 48 hours of drug action in each group, the cell status was observed under a microscope. The cell death in the Docetaxel/Eribulin single drug group was obvious, and the cell death in the combined drug group was the most significant. The cell viability in the control group was better, and the cell death in the Defactinib single drug group was more significant. Flow cytometry analysis results showed that the positive rates of CRT and Annexin V in the combined drug group were significantly higher than those in the single drug group. The assay results of Docetaxel are shown in Figs. 14a and 14b; the assay results of Eribulin are shown in Figs. 15a and 15b.

### Example 9: Study on the in vivo antitumor effect of docetaxel injection in subcutaneous allograft tumor model of breast cancer 4T1 cells in BALB/c mice

### Test Materials:

Mice: Female BALB/c mice aged 6-8 weeks were purchased from SHANGHAI SLAC LABORATORY ANIMAL CO. LTD. The assay began after the animals were adapted to the assay environment upon arrival. The animals were kept in IVC (independent ventilation system) cages (5 per cage) in an SPF animal room. All cages, padding and drinking water must be sterilized before use. All assay personnel should wear protective clothing and latex gloves when operating in the animal room. Cages, feed and drinking water were replaced twice a week. The feeding environment and lighting conditions were as follows:
Temperature: 20-26 °C
Humidity: 40-70%
Lighting period: 12 hours light, 12 hours no light
Cages: Made of polycarbonate, 300 mm × 180 mm × 150 mm. The padding was corn cobs, which was replaced twice a week.
Food: The assay animals had free access to food (dry pelleted food sterilized by irradiation) throughout the assay period.
Drinking water: Assay animals were free to drink sterile water.
Cage identification: The animal information card for each cage should indicate the number of animals in the cage, gender, strain, receipt date, dosing regimen, assay number, group and start date of the assay.
Animal identification: Assay animals were identified with ear tags.

Compound information is shown in Table 8

T

**able 8:**

| Drug Name | Concentration (mg / mL) | Store conditi on | Source | Solvent | Total |
|---|---|---|---|---|---|
| Docetaxel injection (Docetaxel/DTX) | 20mg: 0.5ml | 4 °C | Jiangsu Hengrui Medicine Co., Ltd. | PBS | 50 mg |
| IN10018 | / | 4 °C | Synthesized according to the method in patent WO2010058032 | DPBS | 190 mg |
| MAX10181 (small molecule PDL1 inhibitor) | / | Room temper ature | MaxiNovel Pharmaceuticals Co.,Ltd. (DCG035H-180901(ST EP6.105)-STD) | 10% KolliphorRH40 +20%SBE--CD+ 70%Water | 2730 mg |
| Polyoxyethylene 40 hydrogenated castor oil (Kolliphor RH40) | / | Room temper ature | BASF(14922168EO) | / | / |
| Sulfobutyl-β-cyclodex trin sodium salt (SBE-β-CD) | / | Room temper ature | Aladdin (C125030) | / | / |

Breast cancer cell 4T1 (source: Nanjing Cobioer Biotechnology Co., Ltd., catalog number: CBP60352) was maintained and passaged by INXMED (Nanjing) Co., Ltd. The cells were cultured in a monolayer in vitro in RPMI-1640 medium with 10% fetal bovine serum in a 37 °C, 5% CO₂ incubator. Trypsin-EDTA was used for routine digestion and passage treatment two to three times a week. When the cells were in the exponential growth phase and the adherent confluence reached 80%-90%, the cells were harvested and plated.

### Cell inoculation and grouping

0.1 mL of cell suspension containing 2 x 10⁵ cells was subcutaneously inoculated on the right back of each mouse. When the tumor volume reached ~52 mm³ (10th day after cell inoculation), the mice were randomly divided into groups for drug administration according to the tumor volume. The group information is shown in Table 9.

**Table 9. Dosing regimen of the test substance in 4T1 mouse transplant tumor model**

| Group | Compound treatment | Number of animal¹ | Dosage (mg/kg) | Dosing volume parameters (mL/kg)² | Route of administrat ion | Dosing frequency |
|---|---|---|---|---|---|---|
| 1 | Control group | 6 | N/A | 10 | *p.o*. | BID*21 days |
| 2 | DTX | 6 | 10 | 10 | *i.p.* | BIW*3 weeks |
| 3 | MAX10181 | 6 | 120 | 10 | *p.o*. | BID*21 days |
| 4 | DTX+IN10018 | 6 | 10+25 | 10 | *i.p. + p.o.* | BIW*3 weeks+QD*21 days |
| 5 | DTX+MAX101 81 | 6 | 10+120 | 10 | *i.p. + p.o.* | BIW*3 weeks+BID*21 days |
| 6 | DTX+IN10018+ MAX10181 | 6 | 10+25+1 20 | 10 | *i.p. + p.o. + p.o*. | BIW*3 weeks+QD*21 days+BID*21 days |

| | | | | | | |
|---|---|---|---|---|---|---|
| Note: 1. Number of mice in each group; 2. Dosing volume: 10 mL/kg based on mouse body weight. If the body weight dropped by more than 15%, the administration of the animals would be stopped; When the body weight recovered to 90%, the administration would be resumed. | | | | | | |

The formulation of the test substances is detailed in Table 10.

**Table 10.**

| Drug Name | Dosage (mg/kg) | Concentrat ion (mg/mL) | Formulation process |
|---|---|---|---|
| Control group (10% KolliphorRH40+20%SB E-β-CD+70%Water) | / | / | 7.2g of SBE-β-CD was weighed and dissolved in 32.4ml of ultrapure water. The mixture was vortexed and ultrasonically mixed. Then 3.6ml of KolliphorRH40 was added. The mixture was mixed by pipetting. The formulation was prepared once every 4 days. |
| DTX | 10 | 1 | To a bottle of docetaxel stock solution (0.5 ml) was added 1.5 ml of docetaxel special solvent and the mixture was mixed well. The concentration was 10 mg/ml. Then 0.7 ml of the above solution was taken out and 6.3 ml of DPBS was added. The mixture was mixed well by pipetting. The formulation was prepared immediately before use. |
| MAX10181 | 120 | 12 | 432 mg of MAX10181 was weighed and 36 mL of the prepared control solution was added. The mixture was vortexed and sonicated to obtain a stable and uniform milky white solution. The formulation was prepared once every 4 days. |
| IN10018 | 25 | 2.5 | 36mg of IN10018 was weighed and diluted by 14.4 mL of DPBS. The mixture was mixed well to obtain a clear solution. |
| | | | The formulation was prepared once every 4 days. |

### Daily observation of assay animals

The formulation and any modifications of this assay protocol had been assessed and approved by the IACUC of ArrayBridge. The use and welfare of assay animals were carried out in accordance with AAALAC regulations. The health status and mortality of animals were monitored daily, with routine checks including observation of tumor growth and the impact of drug treatment on the animals' daily behavior, such as activity, food and water intake (estimated by visual inspection), changes in body weight, physical signs, or any other abnormalities. The number of animal deaths and side effects in each group were recorded based on the number of animals in each group.

### Assay termination

If the animal's health condition continued to deteriorate, or if the tumor volume exceeded 3,000 mm³, or there was a serious disease or pain, euthanasia should be carried out. In the following cases, the veterinarian was notified and euthanasia was performed: obvious weight loss, i.e., weight loss greater than 20%; inability to freely eat and drink; the average tumor volume of the control group reached 2000 mm³, the assay was terminated. Animals showed the following clinical signs and continued to deteriorate: piloerection, hunched back, pale ears, nose, eyes, or feet, rapid breathing, convulsions, continuous diarrhea, dehydration, slow movement, vocalization.

### Tumor measurements and assay parameters

Tumor diameters were measured three times a week using calipers. The tumor volume was calculated using the formula: V = 0.5 × *a × b²,* where *a* and *b* represent the long and short diameters of the tumor, respectively.

Based on the tumor volume on the first day after grouping, the tumor growth inhibition rate TGI (%) was calculated according to the following formula: TGI (%) = [1 - (average tumor volume of a treatment group - average tumor volume of the treatment group at the beginning of treatment) / (average tumor volume of the solvent control group - average tumor volume of the solvent control group at the beginning of treatment)] × 100%.

### Statistical analysis

Statistical analysis was based on tumor volume at the end of the assay using Prism Graphpad software. Comparisons between multiple groups were analyzed using Two-way ANOVA and Fisher's LSD test. A P-value of less than 0.05 was considered statistically significant.

### Assay results

In vivo efficacy of the test substance DTX and/or MAX10181 combined with IN10018 in the BALB/c mouse subcutaneous allograft tumor model of 4T1 mouse breast cancer cells. After cell inoculation, tumor growth was observed every day. On the 10th day after inoculation, mice were grouped based on tumor volume. The average tumor volume at enrollment was approximately 52 mm³. Due to tumor burden, animals in the control group were euthanized on the 31st day after inoculation, which was the 21st day after administration according to the group, and the entire assay was over.

On the 21st day after administration according to the group, the tumor volume of the control group was 2129.2 ± 322.0 mm³. The tumor volumes of the DTX (10 mg/kg), MAX10181 (120 mg/kg), DTX + IN10018 (10 + 25 mg/kg), DTX + MAX10181 (10 +120 mg/kg) and DTX + IN10018 + MAX10181 (10 + 25 + 120 mg/kg) treatment groups were 1818.7 ± 644.0 mm³, 2205.7 ± 912.7 mm³, 1182.9 ± 264.3 mm³, 1480.3 ± 345.6 mm³ and 829.4 ± 249.1 mm³, respectively (see Table 11). Compared with the control group, the tumor volume in the DTX (10 mg/kg), MAX10181 (120 mg/kg), DTX + IN10018 (10 + 25 mg/kg), DTX + MAX10181 (10 + 120 mg/kg) and DTX + IN10018 + MAX10181 (10 + 25 + 120 mg/kg) groups had a tumor inhibition rate TGI of 14.9 % (*p* = 0.0147), -3.7 % (*p* =0.5642), 45.6 % (*p* <0.0001), 31.3 % (*p* <0.0001) and 62.6 % (*p <* 0.0001), respectively (see Table 11). When comparing the overall tumor volume with the three-drug combination group of DTX+IN10018+MAX10181 (10+25+120 mg/kg) for statistical analysis, the P values of the control group, DTX (10 mg/kg), MAX10181 (120 mg/kg), DTX + IN10018 (10 + 25 mg/kg) and DTX + MAX10181 (10 + 120 mg/kg) were *p <* 0.0001, *p <* 0.0001, *p <* 0.0001, *p =* 0.0055 and p< 0.0001, respectively. The tumor volumes of each dose group at different time periods are shown in Fig. 16.

**Table 11: Data on day 21 after administration according to the group**

| Group | Tumor volume on day 0 (mm³) ¹ | Tumor volume on day 21 (mm ³) | TGI (%) | P value² | P value³ |
|---|---|---|---|---|---|
| Control group | 52.4 ± 15.0 | 2129.2 ± 322.0 | / | / | <0.0001 **** |
| DTX | 51.1 ± 13.2 | 1818.7 ± 644.0 | 14.9 | 0.0147 * | <0.0001 **** |
| MAX10181 | 51.7 ± 13.9 | 2205.7 ± 912.7 | -3.7 | 0.5642 | <0.0001 **** |
| DTX+IN10018 | 52.2 ± 10.1 | 1182.9 ± 264.3 | 45.6 | <0.0001 **** | 0.0055 ** |
| DTX+MAX10181 | 52.7 ± 12.1 | 1480.3 ± 345.6 | 31.3 | <0.0001 **** | <0.0001 **** |
| DTX+IN10018+M AX10181 | 52.8 ± 10.4 | 829.4 ± 249.1 | 62.6 | <0.0001 **** | / |

| | | | | | |
|---|---|---|---|---|---|
| Note: 1. Calculated based on the number of days after administration according to the group, data are mean ± standard error. 2. *: p<0.05, ****: p<0.0001, vs. control group, Two-way ANOVA. 3. **: p<0.01, ****: p<0.0001, vs. DTX+IN10018+MAX10181 (10+25+120 mg/kg) group, Two-way ANOVA. | | | | | |

The assay was carried out according to the dosing regimen. During the assay, the animals' activities such as eating and drinking were observed every day, and the body weight of the animals was recorded three times a week. The animal weight curve is shown in Fig. 17. During the entire dosing cycle, animals in the MAX10181-related groups had diarrhea, which led to the death of one animal in G3 MAX10181 (120 mg/kg) and G5 DTX+MAX10181 (10+120 mg/kg), respectively. In addition, the body weight of DTX + IN10018+MAX10181 (10 + 25+120 mg/kg) decreased, but decrease did not exceed 10%; the animals in each group were in good mental and motor states and tolerated various modes of administration.

### Conclusion

Compared with the blank control group, the treatment groups of DTX (10 mg/kg), DTX + IN10018 (10+25 mg/kg), DTX+MAX10181 (10+120 mg/kg) and DTX + IN10018+MAX10181 (10+25+120 mg/kg) all had significant tumor growth inhibition effects, with statistical differences compared to the control group. Throughout the entire dosing period, the tumor volume of the DTX + IN10018 (10 + 25 mg/kg) group was smaller than that of the DTX (10 mg/kg) single-drug group, indicating that the combination of DTX and IN10018 had better efficacy than that of the single drug; the tumor volume of the DTX + IN10018+MAX10181 (10 + 25+120 mg/kg) three-drug combination group was relative to that of each single-drug group and each two-drug combination group, and was statistically different from those of the other groups, indicating that the combination of DTX, IN10018 and MAX10181 had a better effect in inhibiting tumor growth. Although the administration of MAX10181 to the relevant groups caused diarrhea in the animals, and resulted in the death of one animal in each of the MAX10181 (120 mg/kg) and DTX+MAX10181 (10+120 mg/kg) groups, the mental and motor conditions of the remaining animals were good, indicating that the animals showed a certain tolerance to the combination of DTX + IN10018+MAX10181 (10 + 25+120 mg/kg).

### Example 10: Study on the Induction of Immunogenic Cell Death Targets of Mouse Breast Cancer 4T1 Cells in Vitro by Docetaxel and AMP945.

### Assay materials:

### 1) Drugs used in this assay

Docetaxel was provided by MCE, Lot No.: 111613.

AMP945 was provided by MCE, Lot No.: 143253.

### 2) Antibodies used in this assay

Recombinant Alexa Fluor^{®} 647 fluorescent Anti-Calreticulin antibody (Abcam, Cat No.: ab196159, Lot No.: CR33676773). Annexin V-apoptosis detection kit (Beyotime, Cat No.: C1062L, Lot No.: 122221220706).

### Assay method:

4T1 cells (from Nanjing Cobioer Biotechnology Co., Ltd., article number: CBP60352) were cultured with RPMI-1640 (Shanghai Basalmedia, Cat No.: L210KJ, Lot No.: F210916)+10%FBS (Gibco, Cat No.: 10099-141c, Lot No.: 2158737cp), wherein the culture conditions were 37 °C and 5% CO₂. Trypsin was used for routine digestion and passage treatment two or three times a week. When the cells were in exponential growth period and the adherent confluence reached 80%-90%, the cells were collected and plated. 4T1 cells were digested with trypsin. The cells were then collected and counted. According to the counting results, the cells were diluted with RPMI-1640+10%FBS, and the dilution concentration was 50,000 cells per ml. Then, the cells were plated on 12-well culture plates, and 2 ml of cell suspension (100,000 cells) was laid in each well. After plating, the cells were cultured in 37 °C and 5% CO₂ incubator. After the cells were spread for 24 hours, six groups were set up, wherein the first group was a control group, and the culture medium was added; the second group was AMP945 with a concentration of 3µM; the third group was AMP945 with a concentration of 6 µM; the fourth group was Docetaxel with a concentration of 0.3 µM; the fifth group was AMP945(3 µM) combined with Docetaxel (0.3 µM); the sixth group was AMP945(6 µM) combined with Docetaxel (0.3 µM). The drugs were mixed and the mixture was cultured in a 5%CO₂ incubator at 37°C for 48 hours.

### Assay result

After 48 hours of drug action, the cells were collected for flow cytometry. The cells were washed twice with flow buffer (PBS+2%FBS). 0.5 µl of AF647 anti-Calreticulin antibody (abcam) was added to each well, and mixed. The cells were incubated at 4°C in the dark. After 20 min of incubation, the flow buffer was added. Annexin staining kit (Beyotime) was used. 195 µl of Annexin-V-FITC binding solution was added and mixed with the cells by pipetting. 5 µl of Annexin-V-FITC antibody was added, and gently mixed. Finally, 10 µl of PI dye was added and mixed, and the cells were incubated in the dark at room temperature for 15 min. The sample was sent to a flow cytometer for signal determination.

The results of flow cytometry analysis showed that the CRT positive rate and the Annexin-V positive rate in the two-drug combination group were significantly better than those in the single drug group and the control group, as shown in Figs. 18a and 18b.

### Example 11: Study on the induction of Immunogenic Cell Death Targets of Mouse Breast Cancer 4T1 Cells in vitro by Eribulin and AMP945.

### Assay materials:

### 1) Drugs used in this assay

Eribulin was provided by Beijing Chempion Biotechnology Co., Ltd, Lot No.: HG-000000011-000-001.

AMP945 was provided by MCE, Lot No.: 143253.

### 2) Antibodies used in this assay

Recombinant Alexa Fluor^{®} 647 fluorescent Anti-Calreticulin antibody (Abcam, Cat No.: ab196159, Lot No.: CR33676773). Annexin V-apoptosis detection kit (Beyotime, Cat No.: C1062L, Lot No.: 122221220706).

### Assay method:

4T1 cells (from Nanjing Cobioer Biotechnology Co., Ltd., article number: CBP60352) were cultured with RPMI 1640 (Shanghai Basalmedia, Cat No.: L210KJ, Lot No.: F210916)+10%FBS (Gibco, Cat No.: 10099-141c, Lot No.: 2158737cp), wherein the culture conditions were 37 °C and 5% CO₂. Trypsin was used for routine digestion and passage treatment two or three times a week. When the cells were in exponential growth period and the adherent confluence reached 80%-90%, the cells were collected and plated. 4T1 cells were digested with trypsin. The cells were then collected and counted. According to the counting results, the cells were diluted with RPMI-1640+10%FBS, and the dilution concentration was 50,000 cells per ml. Then, the cells were plated on 12-well culture plates, and 2 ml of cell suspension (100,000 cells) was laid in each well. After plating, the cells were cultured in 37 °C and 5% CO₂ incubator. After the cells were spread for 24 hours, six groups were set up, wherein the first group was a control group, and the culture medium was added; the second group was AMP945 with a concentration of 3µM; the third group was AMP945 with a concentration of 6 µM; the fourth group was Eribulin with a concentration of 0.3 µM; the fifth group was AMP945(3 µM) combined with Eribulin (0.3 µM); the sixth group was AMP945(6 µM) combined with Eribulin (0.3 µM). The drugs were mixed and the mixture was cultured in a 5%CO₂ incubator at 37°C for 48 hours.

### Assay result

After 48 hours of drug action, the cells were collected for flow cytometry. The cells were washed twice with flow buffer (PBS+2%FBS). 0.5 µl of AF647 anti-Calreticulin antibody (abcam) was added to each well, and mixed. The cells were incubated at 4°C in the dark. After 20 min of incubation, the flow buffer was added. Annexin staining kit (Beyotime) was used. 195 µl of Annexin-V-FITC binding solution was added and mixed with the cells by pipetting. 5 µl of Annexin-V-FITC antibody was added, and gently mixed. Finally, 10 µl of PI dye was added and mixed, and the cells were incubated in the dark at room temperature for 15 min. The sample was sent to a flow cytometer for signal determination.

The results of flow cytometry analysis showed that the CRT positive rate and the Annexin-V positive rate in the two-drug combination group were significantly better than those in the single drug group and the control group, as shown in Figs. 19a and 19b.

All references mentioned in the present disclosure are incorporated by reference in their entirety as if each document was listed separately. It should be understood that after reading the present disclosure, those skilled in the art can make various changes or modifications to the present disclosure, and these equivalents also fall within the scope defined by the claims of this application.

## Claims

1. Use of an FAK inhibitor, a microtubule inhibitor and an immune checkpoint inhibitor in the manufacture of a medicament for treating a tumor in a subject.

2. A pharmaceutical combination product of an FAK inhibitor, a microtubule inhibitor and an immune checkpoint inhibitor for use in treating a tumor in a subject.

3. A method for treating a tumor, comprising administering to a subject a therapeutically effective amount of an FAK inhibitor, a microtubule inhibitor and an immune checkpoint inhibitor.

4. The use, the pharmaceutical combination product, or the method according to any one of claims 1-3, wherein the FAK inhibitor and the microtubule inhibitor induce immunogenic cell death (ICD).

5. The use, the pharmaceutical combination product, or the method according to any one of claims 1-4, wherein the FAK inhibitor is IN10018, Defactinib, GSK2256098, PF-00562271, VS-4718, APG-2449, AMP945, AMP886, or a pharmaceutically acceptable salt thereof, alternatively, the FAK inhibitor is IN10018, Defactinib, AMP945, or a pharmaceutically acceptable salt thereof, and yet alternatively, the FAK inhibitor is IN10018, or a pharmaceutically acceptable salt thereof, alternatively, the FAK inhibitor is IN10018 tartrate, wherein the IN10018 has a structure of:

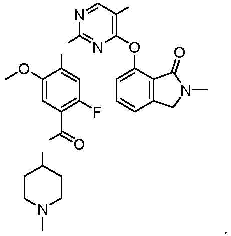

6. The use, the pharmaceutical combination product, or the method according to any one of claims 1-5, wherein the microtubule inhibitor is a taxane, Eribulin, Ixempra or a vinca alkaloid.

7. The use, the pharmaceutical combination product, or the method according to any one of claims 1-6, wherein the microtubule inhibitor is a taxane.

8. The use, the pharmaceutical combination product, or the method according to any one of claims 6-7, wherein the taxane is Docetaxel, Paclitaxel, Cabazitaxel or Cephalomannine, alternatively Docetaxel or Paclitaxel.

9. The use, the pharmaceutical combination product, or the method according to any one of claims 1-8, wherein the microtubule inhibitor is a Docetaxel.

10. The use, the pharmaceutical combination product, or the method according to any one of claims 1-8, wherein the microtubule inhibitor is Paclitaxel.

11. The use, the pharmaceutical combination product, or the method according to any one of claims 1-6, wherein the microtubule inhibitor is Eribulin.

12. The use, the pharmaceutical combination product, or the method according to any one of claims 1-11, wherein the immune checkpoint inhibitor is an anti-PD-1/PD-L1 antibody, a PD-1/PD-L1 small molecule inhibitor, or a TIGIT inhibitor.

13. The use, the pharmaceutical combination product, or the method according to any one of claims 1-12, wherein the immune checkpoint inhibitor is an anti-PD-1/PD-L1 antibody, optionally, the anti-PD-1/PD-L1 antibody is Pembrolizumab, Tislelizumab, Nivolumab, Toripalimab, Atezolizumab, Durvalumab, Avelumab, Camrelizumab, Sintilimab, Cemiplimab, Envafolimab, BMS-936559, JS003, SHR-1316, GS-4224, AN-4005, or MX-10181.

14. The use, the pharmaceutical combination product, or the method according to any one of claims 1-12, wherein the immune checkpoint inhibitor is a PD-1/PD-L1 small molecule inhibitor, optionally, the PD-1/PD-L1 small molecule inhibitor is INCB-086550, Lazertinib, IMMH-010, CA-170, ABSK043, or RRx-001.

15. The use, the pharmaceutical combination product, or the method according to any one of claims 1-12, wherein the immune checkpoint inhibitor is a TIGIT inhibitor, optionally, the TIGIT inhibitor is Ociperlimab (BGB-A1217), Vibostolimab, Domvanalimab (AB154), Tiragolumab, Belrestotug, Etigilimab, ONO-4686, JS-006, AZD-2936, HLX-301, SEA-TGT, M-6223, IBI-939, COM-902, AB-308, AGEN-1777, AK-127, BAT-6021, BAT-6005, ASP-8374, PM-1022, BMS-986207, HB0036, or IBI-321.

16. The use, the pharmaceutical combination product, or the method according to any one of claims 1-4, wherein the FAK inhibitor is IN10018 or a pharmaceutically acceptable salt thereof, the microtubule inhibitor is Docetaxel; and the immune checkpoint inhibitor is an anti-PD-1/PD-L1 antibody, or a PD-1/PD-L1 small molecule inhibitor, alternatively, the FAK inhibitor is IN10018 or a pharmaceutically acceptable salt thereof, the microtubule inhibitor is Docetaxel; the immune checkpoint inhibitor is an anti-PD-1/PD-L1 antibody.

17. The use, the pharmaceutical combination product, or the method according to any one of claims 1-4, wherein the FAK inhibitor is IN10018 or a pharmaceutically acceptable salt thereof, the microtubule inhibitor is Paclitaxel, and the immune checkpoint inhibitor is an anti-PD-1/PD-L1 antibody, or a PD-1/PD-L1 small molecule inhibitor, alternatively, the FAK inhibitor is IN10018 or a pharmaceutically acceptable salt thereof, the microtubule inhibitor is Paclitaxel; the immune checkpoint inhibitor is an anti-PD-1/PD-L1 antibody.

18. The use, the pharmaceutical combination product, or the method according to any one of claims 1-17, wherein the FAK inhibitor, the microtubule inhibitor and the immune checkpoint inhibitor are administered simultaneously or sequentially to the subject.

19. The use, the pharmaceutical combination product, or the method according to any one of claims 1-18, wherein the tumor is bladder cancer, breast cancer, cervical cancer, colon cancer (including colorectal cancer), esophageal cancer, esophageal squamous cell carcinoma, head and neck cancer, liver cancer, lung cancer (including small cell lung cancer and non-small cell lung cancer), melanoma, myeloma, rhabdomyosarcoma, inflammatory myofibroblastoma, neuroblastoma, pancreatic cancer, prostate cancer, kidney cancer, renal cell carcinoma, sarcoma (including osteosarcoma), skin cancer (including squamous cell carcinoma), gastric cancer, testicular cancer, thyroid cancer, uterine cancer, mesothelioma, cholangiocarcinoma, leiomyosarcoma, liposarcoma, nasopharyngeal carcinoma, neuroendocrine carcinoma, ovarian carcinoma, salivary adenocarcinoma, metastases caused by spindle cell carcinoma, anaplastic large cell lymphoma, undifferentiated thyroid cancer, non-Hodgkin's lymphoma, Hodgkin's lymphoma, glioma or hematologic malignancies such as acute myeloid leukemia (AML), acute lymphocytic leukemia (ALL), diffuse large B-cell lymphoma (DLBCL), follicular lymphoma (FL), chronic lymphocytic leukemia (CLL), or chronic myelogenous leukemia (CML); alternatively, the tumor is breast cancer, ovarian cancer, colon cancer (including colorectal cancer), lung cancer (including small cell lung cancer and non-small cell lung cancer), melanoma, or pancreatic cancer.

20. The use, the pharmaceutical combination product, or the method according to any one of claims 1-19, wherein the tumor is breast cancer, ovarian cancer, or colon cancer (including colorectal cancer).

21. A kit or pharmaceutically acceptable composition, comprising:
(a) an FAK inhibitor;
(b) a microtubule inhibitor; and
(c) an immune checkpoint inhibitor.

22. The kit or composition according to claim 21, wherein the FAK inhibitor and the microtubule inhibitor induce immunogenic cell death (ICD).

23. The kit or composition according to any one of claims 21-22, wherein the FAK inhibitor is IN10018, Defactinib, GSK2256098, PF-00562271, VS-4718, APG-2449, AMP945, AMP886, or a pharmaceutically acceptable salt thereof, alternatively, the FAK inhibitor is IN10018, Defactinib, AMP945, or a pharmaceutically acceptable salt thereof, and yet alternatively, the FAK inhibitor is IN10018, or a pharmaceutically acceptable salt thereof, alternatively, the FAK inhibitor is IN10018 tartrate, wherein the IN10018 has a structure of:

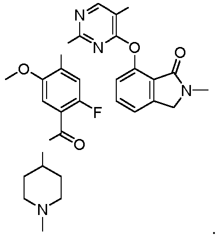

24. The kit or composition according to any one of claims 21-23, wherein the microtubule inhibitor is a taxane, Eribulin, Ixempra or a vinca alkaloid.

25. The kit or composition according to any one of claims 21-24, wherein the microtubule inhibitor is a taxane.

26. The kit or composition according to any one of claims 24-25, wherein the taxane is Docetaxel, Paclitaxel, Cabazitaxel or Cephalomannine, alternatively Docetaxel or Paclitaxel.

27. The kit or composition according to any one of claims 21-26, wherein the microtubule inhibitor is a Docetaxel.

28. The kit or composition according to any one of claims 21-26, wherein the microtubule inhibitor is Paclitaxel.

29. The kit or composition according to any one of claims 21-24, wherein the microtubule inhibitor is Eribulin.

30. The kit or composition according to any one of claims 21-29, wherein the immune checkpoint inhibitor is an anti-PD-1/PD-L1 antibody, a PD-1/PD-L1 small molecule inhibitor, or a TIGIT inhibitor.

31. The kit or composition according to any one of claims 21-30, wherein the immune checkpoint inhibitor is an anti-PD-1/PD-L1 antibody, optionally, the anti-PD-1/PD-L1 antibody is Pembrolizumab, Tislelizumab, Nivolumab, Toripalimab, Atezolizumab, Durvalumab, Avelumab, Camrelizumab, Sintilimab, Cemiplimab, Envafolimab, BMS-936559, JS003, SHR-1316, GS-4224, AN-4005, or MX-10181.

32. The kit or composition according to any one of claims 21-30, wherein the immune checkpoint inhibitor is a PD-1/PD-L1 small molecule inhibitor, optionally, the PD-1/PD-L1 small molecule inhibitor is INCB-086550, Lazertinib, IMMH-010, CA-170, ABSK043, or RRx-001.

33. The kit or composition according to any one of claims 21-30, wherein the immune checkpoint inhibitor is a TIGIT inhibitor, optionally, the TIGIT inhibitor is Ociperlimab (BGB-A1217), Vibostolimab, Domvanalimab (AB154), Tiragolumab, Belrestotug, Etigilimab, ONO-4686, JS-006, AZD-2936, HLX-301, SEA-TGT, M-6223, IBI-939, COM-902, AB-308, AGEN-1777, AK-127, BAT-6021, BAT-6005, ASP-8374, PM-1022, BMS-986207, HB0036, or IBI-321.

34. The kit or composition according to any one of claims 21-22, wherein the FAK inhibitor is IN10018 or a pharmaceutically acceptable salt thereof, the microtubule inhibitor is Docetaxel; and the immune checkpoint inhibitor is an anti-PD-1/PD-L1 antibody, or a PD-1/PD-L1 small molecule inhibitor, alternatively, the FAK inhibitor is IN10018 or a pharmaceutically acceptable salt thereof, the microtubule inhibitor is Docetaxel; the immune checkpoint inhibitor is an anti-PD-1/PD-L1 antibody.

35. The kit or composition according to any one of claims 21-22, wherein the FAK inhibitor is IN10018 or a pharmaceutically acceptable salt thereof, the microtubule inhibitor is Paclitaxel, and the immune checkpoint inhibitor is an anti-PD-1/PD-L1 antibody, or a PD-1/PD-L1 small molecule inhibitor, alternatively, the FAK inhibitor is IN10018 or a pharmaceutically acceptable salt thereof, the microtubule inhibitor is Paclitaxel; the immune checkpoint inhibitor is an anti-PD-1/PD-L1 antibody.

36. The kit or composition according to any one of claims 21-35, wherein the kit or composition is used as a medicament.

37. The kit or composition according to any one of claims 21-36, wherein the medicament is used for treating a tumor, wherein the tumor is bladder cancer, breast cancer, cervical cancer, colon cancer (including colorectal cancer), esophageal cancer, esophageal squamous cell carcinoma, head and neck cancer, liver cancer, lung cancer (including small cell lung cancer and non-small cell lung cancer), melanoma, myeloma, rhabdomyosarcoma, inflammatory myofibroblastoma, neuroblastoma, pancreatic cancer, prostate cancer, kidney cancer, renal cell carcinoma, sarcoma (including osteosarcoma), skin cancer (including squamous cell carcinoma), gastric cancer, testicular cancer, thyroid cancer, uterine cancer, mesothelioma, cholangiocarcinoma, leiomyosarcoma, liposarcoma, nasopharyngeal carcinoma, neuroendocrine carcinoma, ovarian carcinoma, salivary adenocarcinoma, metastases caused by spindle cell carcinoma, anaplastic large cell lymphoma, undifferentiated thyroid cancer, non-Hodgkin's lymphoma, Hodgkin's lymphoma, glioma or hematologic malignancies such as acute myeloid leukemia (AML), acute lymphocytic leukemia (ALL), diffuse large B-cell lymphoma (DLBCL), follicular lymphoma (FL), chronic lymphocytic leukemia (CLL), or chronic myelogenous leukemia (CML); alternatively, the tumor is breast cancer, ovarian cancer, colon cancer (including colorectal cancer), lung cancer (including small cell lung cancer and non-small cell lung cancer), melanoma, or pancreatic cancer.

38. The kit or composition according to claim 37, wherein the tumor is breast cancer, ovarian cancer, or colon cancer (including colorectal cancer).

39. An FAK inhibitor for use in enhancing immunogenic cell death induced by a microtubule inhibitor in the treatment of a tumor.

40. The FAK inhibitor according to claim 39, wherein the FAK inhibitor is IN10018, Defactinib, GSK2256098, PF-00562271, VS-4718, APG-2449, AMP945, AMP886, or a pharmaceutically acceptable salt thereof, alternatively, the FAK inhibitor is IN10018, Defactinib, AMP945, or a pharmaceutically acceptable salt thereof, alternatively, the FAK inhibitor is IN10018, or a pharmaceutically acceptable salt thereof, alternatively, the FAK inhibitor is IN10018 tartrate, wherein the IN10018 has a structure of:

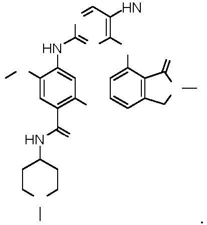

41. The FAK inhibitor according to claim 39 or 40, wherein the microtubule inhibitor is a taxane, Eribulin, Ixempra or vinca alkaloid.

42. The FAK inhibitor according to claim 41, wherein the taxane is Docetaxel, Paclitaxel, Cabazitaxel or Cephalomannine, alternatively Docetaxel or Paclitaxel.

43. The FAK inhibitor according to any one of claims 39-42, wherein the tumor is bladder cancer, breast cancer, cervical cancer, colon cancer (including colorectal cancer), esophageal cancer, esophageal squamous cell carcinoma, head and neck cancer, liver cancer, lung cancer (including small cell lung cancer and non-small cell lung cancer), melanoma, myeloma, rhabdomyosarcoma, inflammatory myofibroblastoma, neuroblastoma, pancreatic cancer, prostate cancer, kidney cancer, renal cell carcinoma, sarcoma (including osteosarcoma), skin cancer (including squamous cell carcinoma), gastric cancer, testicular cancer, thyroid cancer, uterine cancer, mesothelioma, cholangiocarcinoma, leiomyosarcoma, liposarcoma, nasopharyngeal carcinoma, neuroendocrine carcinoma, ovarian carcinoma, salivary adenocarcinoma, metastases caused by spindle cell carcinoma, anaplastic large cell lymphoma, undifferentiated thyroid cancer, non-Hodgkin's lymphoma, Hodgkin's lymphoma, glioma or hematologic malignancies such as acute myeloid leukemia (AML), acute lymphocytic leukemia (ALL), diffuse large B-cell lymphoma (DLBCL), follicular lymphoma (FL), chronic lymphocytic leukemia (CLL), or chronic myelogenous leukemia (CML); alternatively, the tumor is breast cancer, ovarian cancer, colon cancer (including colorectal cancer), lung cancer (including small cell lung cancer and non-small cell lung cancer), melanoma, or pancreatic cancer; still alternatively, the tumor is breast cancer, ovarian cancer, or colon cancer (including colorectal cancer).
